# FASCICULE DE BREVET EUROPEEN

(11) **EP 1 684 792 B1**
(45) Date de publication et mention de la délivrance du brevet: **14.12.2011**
(21) Numéro de dépôt: 04805850.7
(22) Date de dépôt: 19.11.2004
(51) Int. Cl.: A61K 38/21, A61K 9/10

(54) **FORMULATIONS PHARMACEUTIQUES POUR LA LIBERATION PROLONGEE D'INTERLEUKINES ET LEURS APPLICATIONS THERAPEUTIQUES**
PHARMAZEUTISCHE FORMULIERUNGEN FÜR DIE VERZÖGERTE FREISETZUNG VON INTERLEUKONEN UND IHRE THERAPEUTISCHEN ANWENDUNGEN
PHARMACEUTICAL FORMULATIONS FOR THE SUSTAINED RELEASE OF INTERLEUKINS AND THERAPEUTIC APPLICATIONS THEREOF

(30) Priorité: 21.11.2003 FR 0350888
(43) Date de publication de la demande: 02.08.2006
(73) Titulaire: FLAMEL TECHNOLOGIES, 69200 Vénissieux (FR)
(72) Inventeur: BIGNON, Sophie, F-69001 Lyon (FR); MEYRUEIX, Rémi, F-69009 Lyon (FR); SOULA, Olivier, F-69330 Meyzieu (FR)
(74) Mandataire: Fleurance, Raphaël
(86) Numéro de dépôt international: PCT/FR2004/050607
(87) Numéro de publication internationale: WO 2005/051418

(56) Documents cités:
- EP-A- 0 734 720
- WO-A-00/30618
- WO-A-99/18142
- WO-A-02/078677
- WO-A-03/090727
- WO-A-03/104303
- WO-A-2004/013206
- WO-A-2004/060968
- FR-A- 2 801 226

## Description

La présente invention concerne de nouvelles formulations pharmaceutiques à base de suspensions colloïdales aqueuses stables et fluides pour la libération prolongée de principes actifs protéiniques, à savoir les interleukines (IL), ainsi que les applications thérapeutiques, de ces formulations. Ces formulations pharmaceutiques actives concernent aussi bien la thérapeutique humaine que vétérinaire.

Les interleukines sont un groupe de protéines qui entrent dans la famille des cytokines. Elles présentent de nombreuses activité qui régulent la réponse inflammatoire et la réponse immunologique. Cependant, leur rôle majeur reste l'activation et l'induction de la prolifération des lymphocytes T. Parmi les membres importants de cette famille, on peut citer: IL-1, IL-2, IL-11 et IL-12. Par exemple, IL-2 est produite par les lymphocytes T activés par un antigène. Cette IL-2 est destinée à stimuler les autres lymphocytes T pour permette leur activation et leur différentiation et ainsi moduler la réponse immunitaire à médiation cellulaire.

Les interleukines sont utilisés en thérapeutique, mais leur toxicité bien connue reste souvent la cause majeure d'interruption des traitements. Par exemple, dans le cas de l'IL-2, les événements majeurs observés au cours de l'utilisation clinique d'IL-2 sont la fièvre, la nausée, des diarrhées, des réactions cutanées, les douleurs articulaires, une apathie. Dans certains cas, cela nécessite une hospitalisation en réanimation, et il est à noter que dans ces cas rares l'injection d'IL-2 a été mise en cause dans la mortalité de patients.

Outre la toxicité des IL, un autre facteur à prendre en compte pour la libération prolongée de ces protéines thérapeutiques que sont les interleukines, est la nécessité de reproduire au mieux chez le patient une concentration plasmatique en protéine proche de la valeur observée chez le sujet sain.

Cet objectif se heurte à la faible durée de vie des IL dans le plasma, ce qui oblige de manière très contraignante, à les injecter de façon répétée. La concentration plasmatique en protéine thérapeutique présente alors un profil « en dents de scie » caractérisé par des pics élevés de concentration et des minima de concentration très bas. Les pics de concentration, très supérieurs à la concentration basale chez le sujet sain ont des effets nocifs très marqués du fait de la toxicité élevée des protéines thérapeutiques telles que les interleukines et plus précisément l'interleukine IL2. Par ailleurs, les minima de concentration sont inférieurs à la concentration nécessaire pour avoir un effet thérapeutique, ce qui entraîne une mauvaise couverture thérapeutique du patient et des effets secondaires graves à long terme.

Aussi, pour reproduire chez le Patient une concentration plasmatique en interleukine proche de la valeur idéale pour le traitement, il importe que la formulation pharmaceutique considérée permette de libérer la protéine thérapeutique sur une durée prolongée, de façon à limiter les variations de concentration plasmatique au cours du temps.

Par ailleurs, cette formulation active doit de préférence satisfaire au cahier des charges suivant, déjà connu de l'homme de l'art :
1 - libération prolongée d'une ou plusieurs interleukines actives et non dénaturées (non modifiées), de sorte que la concentration plasmatique est maintenue au niveau thérapeutique,
2 - forme liquide suffisamment fluide pour être aisément injectable et stérilisable par filtration sur des filtres dont la taille des pores est inférieure ou égale à 0,2 microns,
3 - forme liquide stable,
4 - biocompatibilité et biodégradabilité,
5 - non toxicité,
6 - non immunogénicité,
7 - excellente tolérance locale.

Pour tenter d'atteindre ces objectifs, plusieurs approches ont déjà été proposées dans l'art antérieur.

Dans la première approche, la protéine thérapeutique native est modifiée par greffage covalent d'une ou de plusieurs chaînes de polymère ou encore par greffage covalent d'une protéine telle que l'albumine sérique humaine (HSA). La protéine ainsi modifiée a une moindre affinité pour ses récepteurs et son temps de demi-vie dans la circulation générale augmente considérablement. L'amplitude de la variation de concentration entre les pics et les creux de concentration plasmatique en protéine est ainsi considérablement réduite. Ainsi, la société Cetus propose, dans son brevet US-B-4 766 106, de greffer une chaîne de polyoxyéthylène à l'interleukine 2, afin d'augmenter sa solubilité et durée de vie plasmatique. D'une façon similaire, la société Human Genome Science propose (US-B-5 876 969) de greffer de façon covalente des interleukines à l'albumine de sérum humaine afin d'augmenter leur durée de vie plasmatique. Cette démarche de modification chimique de la protéine thérapeutique présente de façon générale deux inconvénients majeurs. Tout d'abord, la modification irréversible de la protéine, qui n'est plus alors une protéine humaine et peut conduite à long terme à des problèmes de toxicité et d'immunogénicité. Le deuxième inconvénient provient de la perte partielle de bioactivité de l'interleukine IL 2 ainsi modifiée.

Dans une deuxième approche, il a été proposé d'augmenter la durée d'action grâce à des formulations comportant au moins un polymère et un principe actif, liquides à température et atmosphère ambiantes, injectables et devenant plus visqueuses après injection, par exemple sous l'effet d'un changement de pH et/ou de température.

Ainsi dans ce registre, le brevet US-B-6 143 314 divulgue une solution organique polymère à libération contrôlée de PA, formant un implant solide après injection. Cette solution comprend:
○ (A) 10 à 80 % en poids d'un polymère thermoplastique de base, biocompatible, biodégradable et insoluble dans l'eau ou les fluides physiologiques (par exemple PolyLactique et/ou PolyGlycolique) ;
○ (B) un solvant organique, tel que la N-MéthylPyrrolidone se dispersant dans les fluides physiologiques ;
○ (C) un principe actif (PA) ;
○ (D) et enfin 1 à 50 % en poids d'un agent de libération contrôlée constitué par un copolymére bloc de type PolyLactiqueGlycolique / PolyEthylèneGlycol.
Après injection, (B) se disperse ou se dissipe dans les fluides physiologiques. (A) forme un implant encapsulant (C) qui n'est pas lié de façon covalente ni à (A) ni à (D) et qui se libère alors lentement in vivo.
Le principal inconvénient de cette technique est d'utiliser un solvant organique (B), potentiellement dénaturant pour le PA (C) (e.g. protéines thérapeutiques) et toxique pour le patient. En outre, l'hydrolyse in vivo du polymère (A) génère un acide qui peut-conduire à des problèmes de tolérance locale.

Les demandes PCT WO-A-99/18142 & WO-A-00/18821 concernent des solutions aqueuses de polymères qui contiennent un PA sous forme dissoute ou colloïdale, qui sont administrables à des animaux à sang chaud, notamment par injection et qui forment un dépôt de PA (e.g insuline) gélifié in vivo, car la température physiologique est supérieure à leur température de gélification. Le gel ainsi formé libère le PA de façon prolongée. Ces polymères biodégradables particuliers sont des triblocs ABA ou BAB avec A = polylactique-coglycolique (PLAGA) ou polylactique (PLA) et B = PolyEthylèneGlycol. Les températures de transformation liquide ⇒ gel de ces polymères triblocs sont par exemple de 36, 34, 30 et 26 °C. A l'instar des polymères (A) selon l'US-B-6 143 314, L'hydrolyse de ces polymères triblocs ABA ou BAB in vivo conduit à des acides qui peuvent ne pas être correctement tolérés localement

La demande PCT WO-A-98/11874 décrit des formulations pharmaceutiques comprenant un principe actif lipophile, un polymère gélifiant (Gelrite® = gomme gellane désacétylée ou éthylhydroxycellulose) et un surfactant. L'interaction polymère/surfactant et éventuellement la seule présence d'électrolytes tels que des ions Ca⁺⁺ en concentration physiologique s'agissant du polymère Gelrite®, conduit à la formation d'un gel constitué par un agrégat polymère/surfactant, auquel se lie de façon non covalente le principe actif lipophile. Cette formulation est destinée à une administration locale dans un organe cible (oeil e.g.). L'association agrégat/principe actif qui se forme in situ permet la libération lente du principe actif dans l'organe cible.

Une troisième approche mise en oeuvre pour tenter de prolonger la durée d'action d'une protéine tout en conservant sa bioactivité, fût d'utiliser une protéine thérapeutique non dénaturée et de l'incorporer dans des microsphères ou des implants à base de polymères biocompatibles. Cette approche est notamment illustrée par le brevet US-B-6 500 448 et la demande US-A-2003/0133980 qui décrivent une composition à libération prolongée d'hormone de croissance humaine (hGH) dans laquelle, la protéine hormonale, préalablement stabilisée par complexation avec un métal, est ensuite dispensés dans une matrice polymère biocompatible. Le polymère biocompatible est par exemple un poly(lactide), un poly(glycolide) ou un copolymère poly(lactide-co-glycolide). La composition se présente par exemple sous la forme d'une suspension de microsphères dans une solution de carboxyméthylcellulose de sodium. Cette approche présente plusieurs inconvénients : tout d'abord, au cours du procédé de fabrication des microsphères, la protéine est mise au contact de solvants organiques potentiellement dénaturants. En outre, les microsphères sont d'une taille élevée (1 à 1000 microns), ce qui constitue une contrainte en termes d'injection et de stérilisation aisée sur filtres. Enfin, des problèmes de tolérance locale peuvent survenir lors de l'hydrolyse in situ du polymère.

Selon une quatrième approche, ont été développées des formes à libération prolongée do protéine thérapeutique (notamment d'interleukines) constituées par des suspensions liquides de nanoparticules chargées en protéines. Ces dernières ont permis l'administration de la protéine native dans une formulation liquide de faible viscosité.

Selon une première voie de libération prolongée, la suspension nanoparticulaire de libération prolongée est constituée par des suspensions de liposomes dans lesquelles la protéine thérapeutique native non modifiée est encapsulée. Après injection, la protéine est libérée progressivement des liposomes, ce qui prolonge le temps de présence de la protéine dans la circulation générale. Ainsi par exemple Frossen et al, décrivent dans l'article Cancer Res. 43 p 546, 1983 l'encapsulation d'agents anti-néoplastiques dans des liposomes afin d'en accroître l'efficacité thérapeutique. La libération des agents est cependant trop rapide pour obtenir une réelle libération prolongée. La société Liposome Company Inc, dans son brevet US-B-5 399 331 propose d'améliorer le temps de libération in vitro de l'interleukine 2 en la greffant de façon covalente au liposome. On retombe alors dans les travers de la première approche "protéine modifiée" évoquée ci dessus.

Afin de pallier le manque de stabilité des liposomes, tout en gardant les avantages d'une formulation nanoparticulaire liquide et de basse viscosité, la société Flamel Technologies a proposé une deuxième voie de libération prolongée, dans laquelle la protéine thérapeutique est associée à des nanoparticules d'un polymère hydrosoluble "modifié hydrophobe", c'est-à-dire modifié par greffage de groupements hydrophobes. Ce polymère est choisi, en particulier, parmi les polyaminoacides (polyglutamates ou polyaspartates) porteurs de greffons hydrophobes.

Un des intérêts notables de ces polymères modifiés hydrophobes est de s'auto assembler spontanément dans l'eau pour former des nanoparticules.

Un autre intérêt de ces systèmes est que les protéines ou les peptides thérapeutiques s'associent spontanément avec les nanoparticules de polymères modifiés hydrophobes, cette association est non covalente et s'effectue sans avoir recours à un tensioactif ni à un procédé de transformation potentiellement dénaturant. Il ne s'agit pas d'une encapsulation de la protéine dans une microsphère, comme divulgué dans le brevet US-B-6 500 448 et la demande US-A-2003/0133980. De façon totalement différente, ces nanoparticules de copolyaminoacides modifiés hydrophobes adsorbent spontanément les protéines en solution, sans les modifier chimiquement ni les dénaturer et sans leur faire subir des étapes de traitement agressives du type "mise en émulsion" et "évaporation de solvant". Les formulations peuvent être stockées sous forme liquide ou sous forme lyophilisée.

Après injection, par exemple par voie sous cutanée, ces suspensions de nanoparticules chargées en protéines libèrent progressivement la protéine non dénaturée et bioactive in vivo. De telles associations non covalentes principe actif (PA) protéinique / poly[Glu] ou poly[Asp] sont divulguées dans la demande de brevet WO-A-00/30618.

Cette demande décrit notamment des suspensions colloïdales de pH 7,4 comprenant des associations d'insuline humaine avec des nanoparticules de polyglutamate "modifié hydrophobe. Le tableau ci-dessous rend compte des polyaminoacides "modifiés hydrophobes" mis en oeuvre et des taux d'association obtenus dans les exemples du WO-A-00/30618.

| EXEMPLE | POLYMERE | Taux d'association (%) |
|---|---|---|
| 1 | poly[(Glu-O-Na)_{0,63}bloc(Glu-O-méthyle)_{0,37}] | 55 |
| 2 | poly[Glu-O-Na)_{0,66}-bloc-(Glu-O-éthyle)_{0,34}] | 26 |
| 3 | poly(Glu-O-Na)_{0,65}-bloc-(Glu-O-hexadécyle)_{0,35}] | 36 |
| 4 | poly[(Glu-O-Na)_{0,88}-bloc-(Glu-O-dodécyle)_{0,12}] | > 90 |

Ces suspensions colloïdales titrent 1,4 mg/ml d'insuline et 10 mg/ml de polyaminoacide "modifié hydrophobe".

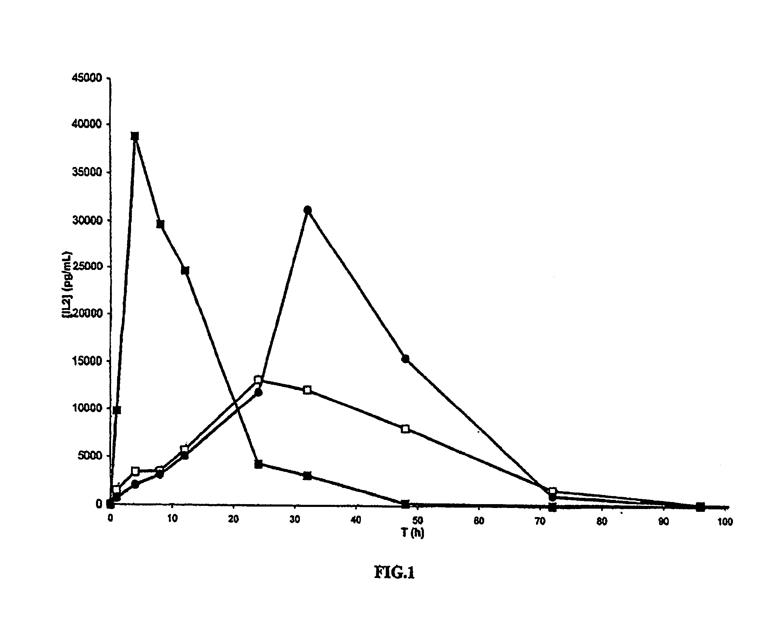

Il ressort de la figure 1 du WO-A-00/30618 que la durée de libération in vivo de l'insuline vectorisée par les suspensions susvisées, est de 12 h. Cette durée de libération gagnerait à être augmentée.

Ainsi, même si cette demande PCT représente déjà un progrès considérable, son contenu technique peut encore être optimisé au regard du cahier des charges énoncé ci-dessus et surtout au regard de l'allongement de la durée de libération in vivo des interleukines.

Les demandes de brevet français non publiées N^{os} 02 07008 du 07/06/2002, 02 09670 du 30/07/2002, 03 50190 du 28/05/2003 et 01 50641 du 03/10/2003, concernent de nouveaux polyaminoacides amphiphiles, hydrosolubles et comprenant des unités acide aspartique et/ou des unités acide glutamique, dans lesquels au moins une partie de ces unités sont porteuses de greffons hydrophobes. A l'instar des polyaminoacides modifiés hydrophobes divulgués dans la demande WO-A-00/30618, ces nouvelles matières premières polymères forment spontanément en milieu liquide aqueux des suspensions colloïdales de nanoparticules qui peuvent être utilisées pour la libération prolongée de PA (insuline). Elles sont biocompatibles, biodégradables et les protéines, en particulier les protéines thérapeutiques s'adsorbent spontanément sur ces nanoparticules sans subir de modification chimique ou de dénaturation.

Ces demandes visent aussi de nouvelles compositions pharmaceutiques, cosmétiques, diététiques ou phytosanitaires à base de ces polyaminoacides.

Les polyaminoacides "modifiés hydrophobes" amphiphiles selon la demande de brevet français N° 02 07008 comprennent des unités acide aspartique et/ou des unités acide glutamique, porteuses de greffons hydrophobes comportant au moins un motif alpha-tocophérol, e.g. : (polyglutamate ou polyaspartate greffé par l'alpha tocophérol d'origine synthétique ou naturelle).

Cette demande non publiée divulgue spécifiquement une suspension colloïdale qui contient des nanoparticules formées par des associations polymère/protéine active et qui est obtenue en mélangeant 1 mg d'un polyglutamate greffé par l'alpha-tocophérol et 7 mg d'insuline dans 1 ml d'eau, à pH 7,0.

Les polyaminoacides "modifiés hydrophobes" amphiphiles selon la demande de brevet français N° 02 09670 comprennent des unités acide aspartique et/ou des unités acide glutamique, porteuses de greffons hydrophobes comportant au moins un motif hydrophobe reliés aux unités acide aspartique et/ou acide glutamiques par l'intermédiaire d'une rotule contenant deux fonctions amides, et plus précisément via un "espaceur" de type lysine ou ornithine.

Cette demande non publiée divulgue spécifiquement une suspension colloïdale qui contient des nanoparticules formées par des associations polymère/protéine active et qui est obtenue en mélangeant 10 mg d'un polyglutamate greffé avec de l'acide palmitique via un "espaceur" lysine et 200 UI d'insuline (7,4 mg) dans 1 ml d'eau, à pH 7,4.

Les polyaminoacides "modifiés hydrophobes" amphiphiles selon la demande de brevet français N° 03 50190 comprennent des unités acide aspartique et/ou des unités acide glutamique, dont certaines sont porteuses d'au moins un greffon relié à une unité acide aspartique ou acide glutamique, par l'intermédiaire d'un "espaceur" "acide aminé" à base de Leu, et/ou ILeu, et/ou Val, et/ou Phe, un groupement hydrophobe en C6-C30 étant relié par une liaison ester à "l'espaceur".

Cette demande non publiée divulgue spécifiquement une suspension colloïdale qui contient des nanoparticules formées par des associations polymère/protéine active et qui est obtenue en mélangeant une solution aqueuse contenant 10 mg d'un polyglutamate greffé avec un greffon -Leu-OC8, -Val-OC12 ou -Val-cholestéryle et 200 UI d'insuline (7,4 mg), par millilitre d'eau, à pH 7,4.

La demande de brevet français N° 01 50641 divulgue des homopolyaminoacides linéaires, amphiphiles, anioniques, comprenant des unités acide aspartique ou des unités acide glutamique dont les extrémités sont porteuses de groupements hydrophobes comportant de 8 à 30 atomes de carbone.

En particulier, les homopolyaminoacides téléchéliques "modifiés hydrophobes" sont par exemple un poly[GluONa] à extrémités PheOC18/C18 ou un poly[GluONa] à extrémités PheOC18/alpha-tocophérol. Cette demande non publiée décrit également une suspension colloïdale qui contient des nanoparticules formées par des associations polymère/protéine active et qui est obtenue en mélangeant 10 mg de l'un des polymères susvisés et 200 UI d'insuline (7,4 mg) par millilitre d'eau, à pH 7,4.

La durée de libération in vivo de l'insuline "vectorisée" par les suspensions selon ces demandes non publiées, gagnerait à être augmentée.

En tout état de cause, tout cet art antérieur sur les suspensions colloïdales de nanoparticules de polyaminoacides modifiés hydrophobes, ne révèle pas de formulation permettant :
(I) d'accroître suffisamment la durée de libération de la protéine active après injection par voie parentérale, en particulier sous cutanée ;
(II) et/ou de réduire le pic de concentration plasmatique de la protéine active après injection de la formulation la contenant.

Dans ces conditions, l'un des objectifs essentiels de la présente invention est donc de proposer une formulation pharmaceutique liquide pour la libération prolongée d'IL(s), remédiant aux carences de l'art antérieur, et en particulier permettant après injection par voie parentérale (e.g. sous cutanée), d'obtenir une durée de libération in vivo prolongée pour des interleukines non dénaturées.

Un autre objectif essentiel de l'invention est de proposer une formulation pharmaceutique liquide à libération prolongée d'interleukine(s) in vivo, qui soit suffisamment fluide pour être aisément injectable et stérilisable par filtration sur des filtres dont la taille des pores est inférieure ou égale à 0,2 microns.

Un autre objectif essentiel de l'invention est de proposer une formulation pharmaceutique liquide à libération prolongée d'interleukine(s) in vivo, qui soit stable à la conservation tant sur le plan physico-chimique que biologique.

Un autre objectif essentiel de invention est de proposer une formulation pharmaceutique liquide à libération prolongée d'interleukine(s) in vivo, qui présente au moins l'une des propriétés suivantes: biocompatibilité, biodégradabilité, atoxicité, non-immunogénicité, bonne tolérance locale.

Un autre objectif essentiel de l'invention est de proposer une formulation pharmaceutique pour la libération prolongée lente d'interleukine(s) in vivo, cette formulation étant une suspension colloïdale aqueuse de basse viscosité comprenant des particules submicroniques de polymère PO auto-associées à au moins une interleukine(s), le polymère PO étant un polymère biodégradable, hydrosoluble et porteur de groupements hydrophobes.

Un autre objectif essentiel de l'invention est de proposer une formulation pharmaceutique de libération prolongée lente d'interleukine(s) in vivo, cette formulation étant une suspension colloïdale aqueuse de basse viscosité comprenant des particules submicroniques de polymères PO auto-associées à au moins une interleukine, le polymère PO étant, par exemple, un polyaminoacide formé par des unités acide aspartique et/ou des unités acide glutamique, au moins une partie de ces unités étant porteuses de greffons comportant au moins un groupement hydrophobe (GH), PO étant en outre biodégradable, hydrosoluble, et amphiphile.

Un autre objectif essentiel de l'invention est de proposer des produits dérivés et/ou des précurseurs de la formulation visée dans les objectifs sus énoncés.

Il est en particulier du mérite de la Demanderesse d'avoir mis au point des formulations pharmaceutiques liquides aqueuses de basse viscosité à température physiologique, qui, de façon surprenante, forment un dépôt gélifié in vivo après administration parentérale aisée chez l'homme ou les mammifères à sang chaud, la formation de ce dépôt n'étant pas déclenchée par un changement de pH, ni de température lors de l'injection parentérale, ni encore de dispersion de solvant organique dans le milieu physiologique. Le dépôt gélifié ainsi formé augmente de façon significative la durée de libération in vivo de l'IL in vivo.

D'où il s'ensuit que l'invention concerne une formulation pharmaceutique liquide pour la libération prolongée d'interleukine(s), cette formulation comprenant au moins une interleukine, éventuellement au moins un autre principe actif (PA), de préférence en solution aqueuse, et une suspension colloïdale, aqueuse, de basse viscosité, à base de particules submicroniques de polymère (PO) biodégradable, hydrosoluble sélectionné dans le groupe consistant en les polyaminoacides, les polysaccharides, les gélatines ou leurs mélanges et porteur de groupements hydrophobes (GH), lesdites particules étant associées de façon non covalente avec ladite interleukine et éventuellement ledit autre principe actif (PA),
caractérisée :
◆ en ce que le milieu dispersif de la suspension est essentiellement constitué par de l'eau,
◆ en ce que sa concentration en [PO] est fixée à une valeur [PO] ≥ 0,9.C1 suffisamment élevée de sorte que ladite formulation pharmaceutique est apte à être injectée par voie parentérale et à former ensuite in vivo un dépôt gélifié, C1 représentant la concentration de "*gélification induite*" des particules de PO telle que mesurée dans un test GI comme décrit ci-après, cette formation de dépôt gélifié :
   ○ étant, d'une part, au moins en partie provoquée par au moins une protéine physiologique présente in vivo,
   ○ et permettant, d'autre part, de prolonger et de contrôler la durée de libération de l'interleukine et éventuellement du PA in vivo, au-delà de 24 h après l'administration,
◆ en ce qu'elle est liquide dans les conditions d'injection,
◆ et en ce qu'elle est également liquide à la température et/ou au pH physiologiques, et/ou en présence :
   * d'électrolyte physiologique en concentration physiologique,
   * et/ou d'au moins un tensioactif.

Avantageusement, cette gélification in vivo ne résulte pas d'un changement de pH et/ou de température, ni d'une dispersion in vivo d'un ou plusieurs solvants organiques éventuellement contenus dans la formulation injectée.

Sans vouloir être lié par la théorie, on peut penser que les protéines physiologiques présentes in vivo dans des concentrations physiologiques, permettent l'agrégation des nanoparticules de PO associées à au moins une interleukine. Une telle gélification s'opère, par exemple, en une ou plusieurs heures, 24 h, 48 h ou 72 h, entre autres.

Selon un mode de définition, qui n'est plus basé sur un comportement in vivo comme ci-dessus indiqué, mais sur un comportement in vitro, l'invention concerne une formulation pharmaceutique liquide pour la libération prolongée d'interleukine(s) et, éventuellement d'autre(s) principe(s) actif(s) -PA-, cette formulation :
○ étant liquide en atmosphère ambiante,
○ étant également liquide à la température et/ou au pH physiologiques et/ou en présence :
   * d'électrolyte physiologique en concentration physiologique,
   * et/ou d'au moins un tensioactif,
○ et comprenant au moins une interleukine, éventuellement au moins un autre principe actif (PA), de préférence en solution aqueuse, et une suspension colloïdale, aqueuse, de basse viscosité, à base de particules submicroniques de polymère PO biodégradable, hydrosoluble sélectionné dans le groupe consistant en les polyaminoacides, les polysaccharides, les gélatines ou leurs mélanges et porteur de groupements hydrophobes GH, lesdites particules étant associées de façon non covalente avec ladite interleukine, de préférence en solution aqueuse, (et éventuellement ledit autre principe actif) et le milieu dispersif de la suspension étant essentiellement constitué par de l'eau,
caractérisée en ce que sa concentration en [PO] est fixée à une valeur suffisamment élevée pour permettre la formation de dépôt gélifié in vitro en présence d'au moins une protéine, ladite concentration en [PO] étant telle que [PO] ≥ 0,9.C1, C1 représentant la concentration de "gélification induite" des particules de PO telle que mesurée dans un test GI comme décrit dans la description.

De préférence, la formulation pharmaceutique liquide selon invention est caractérisée en ce que sa concentration en [PO] est telle que :
■ 20.C1 ≥ [PO] ≥ C1,
■ et mieux encore 10.C1 ≥ [PO] ≥ C1,

Le dépôt gélifié obtenu après injection parentérale de la formulation permet une prolongation intéressante de la durée de libération de la protéine ainsi qu'une réduction du pic de concentration plasmatique d'interleukine(s).

La durée de libération des interleukines est significativement augmentée par rapport à celle des formulations de l'art antérieur, en particulier celles décrites dans la demande de brevet PCT publiée WO-A-00/30618 et les demandes de brevet français non publiées N^{os} 02 07008, 02 09670, 03 50190 et 01 50641.

La prolongation de la durée de libération in vivo induite par les formulations selon l'invention est d'autant plus appréciable que les interleukines libérées sont toujours pleinement bioactives et non dénaturées.

Les interleukines au sens du présent exposé sont indifféremment des interleukines non modifiées ou des interleukines modifiées, par exemple par greffage d'un ou de plusieurs groupements polyoxyéthyléniques. Parmi les protéines de la famille des interleukines, on peut citer: IL-1, IL-2, IL-11, IL-12 et IL 18.

Dans tout le présent exposé, les arrangements supramoléculaires polymère PO associé ou non à au moins une interleukine, et, éventuellement à au moins un autre PA, seront indifféremment désignés par "particules submicroniques" ou "nanoparticules". Cela correspond à des particules de diamètre hydrodynamique moyen (mesuré selon un mode opératoire Md défini infra dans les exemples) e.g. compris entre 1 et 500 nm, de préférence entre 5 et 250 nm.

En outre, il est tout à fait important de noter que ces formulations sont liquides, c'est-à-dire présentent avantageusement une viscosité très faible, qui rend leur injection aisée. Elles ne gélifient qu'in vivo.

Suivant l'invention, les qualificatifs "liquide", "basse" ou "très faible viscosité" correspondent, avantageusement, à une viscosité dynamique à 20 °C inférieure ou égale à 5 Pa.s. La mesure de référence pour la viscosité peut être réalisée, par exemple, à 20 °C à l'aide d'un rhéomètre AR1000 (TA Instruments) équipé d'une géométrie cône-plan (4 cm, 2°). La viscosité v est mesurée pour un gradient de cisaillement de 10 s⁻¹.
Ainsi, la viscosité des formulations selon l'invention peut être, par exemple, comprise entre 1.10⁻³ et 5 Pa.s, de préférence entre 1.10⁻³ et 0,8 Pa.s et, plus préférentiellement encore, entre 1.10⁻³ et 0,5 Pa.s.
Cette faible viscosité rend les formulations de l'invention non seulement aisément injectables par voie parentérale, en particulier par voie intramusculaire ou sous-cutanée, entre autres, mais aussi stérilisables aisément et à moindre coût par filtration sur des filtres de stérilisation de 0,2 µm de taille de pores.
Cet état liquide ou cette faible viscosité des formulations de l'invention existe aussi bien à des températures d'injection correspondant à des températures ambiantes, par exemple comprises entre 4 et 30 °C, qu'à la température physiologique.

La formulation selon l'invention est, de préférence, une suspension colloïdale aqueuse de nanoparticules associés à une ou plusieurs interleukines et éventuellement un ou plusieurs PA. Cela signifie que, conformément à l'invention, le milieu dispersif de cette suspension est essentiellement formé par de l'eau. En pratique, cette eau représente, par exemple, au moins 50 % en poids par rapport à la masse totale de la formulation.

Au sens de l'invention, le terme "protéine" désigne aussi bien une protéine qu'un peptide. Cette protéine ou ce peptide pouvant être modifié ou non, par exemple, par greffage d'un ou de plusieurs groupements polyoxyéthyléniques.

Par "protéines physiologiques", on entend, au sens de l'invention, les protéines et/ou les peptides endogènes des mammifères à sang chaud présents sur le site d'injection.

Par "température physiologique", on entend au sens de l'invention, la température physiologique des mammifères à sang chaud, à savoir par exemple environ 37-42 °C.

Par "pH physiologique", on entend, au sens de l'invention, un pH par exemple compris entre 6 et 7,6.

Par "gel", on entend, au sens de l'invention, un état semi-solide dans lequel se transforme la formulation liquide selon l'invention, et ce spontanément par la seule présence de protéine(s) physiologique(s), sans intervention essentielle du pH physiologique et/ou de la température physiologique et/ou de la présence d'un électrolyte physiologique (Ca⁺⁺ e.g.) et/ou de la dispersion (ou dissipation) in vivo d'un solvant organique éventuellement présent dans la formulation injectée.

Par "électrolyte physiologique", on entend, au sens de l'invention, tout élément électrolyte (par exemple des ions Ca⁺⁺) présent chez les mammifères à sang chaud.

Par "concentration physiologique", on entend, au sens de l'invention, toute concentration physiologique rencontrée chez les mammifères à sang chaud, pour le milieu physiologique considéré.

En outre, les formulations selon l'invention sont non toxiques, bien tolérées localement et stables.

Il est également du mérite des inventeurs d'avoir mis au point un test in vitro GI permettant de sélectionner une population des formulations préférées selon l'invention et de déterminer les concentrations idoines en PO dans les formulations.

Conformément à l'invention, le test GI de mesure de la concentration de gélification Cl, est un test de référence permettant de définir la concentration critique Cl, dénommée ci après concentration de gélification induite Cl, qui caractérise chaque formulation colloïdale selon l'invention.

### Le test GI de détermination de la concentration C1 de gélification induite est le suivant :

Afin de déterminer la concentration Cl, on prépare des formulations colloïdales de concentrations variables en polymère amphiphile selon l'invention et de concentration constante en protéine thérapeutique. A cette fin on met en solution dans de l'eau deionisée des quantités croissantes de poudre sèche du polymère. Les solutions sont maintenues à 25 °C sous agitation magnétique durant 16 heures avant d'être mélangées avec une solution concentrée en protéine thérapeutique. Le volume et la concentration de cette solution de protéine thérapeutique sont ajustés afin d'obtenir la concentration en protéine recherchée pour la formulation [par exemple 2,5 mg/ml d'interleukine 2 (IL2)].

Les formulations colloïdales ainsi préparées sont mélangées à une solution aqueuse d'albumine de sérum bovin (BSA) concentrée à 30 mg/ml, puis centrifugées pendant 15 minutes à 3000 t/min. Les mélanges sont laissés sous agitation douce pendant 24 h avant d'être récupérés pour être caractérisés.

Les mesures de viscoélasticité sont effectuées sur un rhéomètre TA Instruments AR 1000, équipé d'une géométrie cône-plan (diamètre 4cm et angle 1,59). Une déformation de 0,01 rad, située dans le domaine de viscoélasticité linéaire, est imposée de manière sinusoïdale sur une gamme de fréquence comprise entre 0,1 et 300 rad/s. La température de l'échantillon est maintenue constante à 20°C par le biais d'une cellule Peltier.

Les spectres en fréquence du module élastique G' et du module visqueux ou de perte, G", permettent de définir le temps de relaxation caractéristique Tr défini ici comme l'inverse de la fréquence à laquelle le module élastique G' croise le module visqueux G". On trouvera un exposé détaillé de ces questions dans l'ouvrage Ferry, Viscoelastic Properties of Polymers, J.D.Ferry, J.Wiley, NY, 1980 et dans l'article de J. REGALADO et al. Macromolecules 1999, 32, 8580.

La mesure du temps de relaxation Tr en fonction de la concentration en polymère de la formulation permet de définir la concentration C1 à laquelle ce temps Tr excède 1 seconde. Des exemples de valeurs de la concentration de gélification C1 seront donnés dans l'exemple 6 ci après.

De la même façon, on peut définir les concentrations C0,1 et C10 pour lesquelles le temps de relaxation dépasse respectivement 0,1 s et 10 s. Ces concentrations se classant dans l'ordre croissant suivant : C0,1 < C1 < C10.

Suivant une variante de la formulation selon l'invention :
➢ [PO] ≥ C0,1,
➢ de préférence [PO] ≥ C1,
➢ et plus préférentiellement encore [PO] ≥ C10.

Suivant une caractéristique additionnelle avantageuse : [PO] ≤ 20.Cl

Au sens de l'invention et dans tout le présent exposé, les termes "association" ou "associer" employés pour qualifier les relations entre un ou plusieurs principes actifs et les polymères PO (par exemple les polyaminoacides), signifient en particulier que le ou les principes actifs sont liés au(x) polymère(s) PO [par exemple le (ou les) polyaminoacide(s)] par une liaison non covalente, par exemple par interaction électrostatique et/ou hydrophobe et/ou liaison hydrogène et/ou gène stérique.

Les polymères PO selon l'invention sont des polymères biodégradables, hydrosolubles et porteurs de groupements hydrophobes GH. Les groupements hydrophobes peuvent être en nombre réduit vis à vis du reste de la chaîne et peuvent se situer latéralement à la chaîne ou intercalés dans la chaîne, et être répartis de façon aléatoire (copolymère statistique) ou répartis sous forme de séquences ou de greffons (copolymères blocs ou copolymères séquencés).

Sans vouloir se limiter les polymères PO modifiés hydrophobes peuvent être choisis dans le groupe comprenant les copolyaminoacides amphiphiles, les polysaccharides -de préférence dans le sous-groupe comprenant les pullulanes et/ou les chitosans et/ou les mucopolysaceharides-, les gélatines ou leurs mélanges.

Selon un mode préféré de réalisation de l'invention, PO est choisi parmi les copolyaminoacides amphiphiles.
Au sens de l'invention et dans tout le présent exposé, le terme *«polyaminoacide*» couvre aussi bien les oligoaminoacides comprenant de 2 à 20 unités "acide aminé" que les polyaminoacides comprenant plus de 20 unités "acide aminé".

De préférence, les polyaminoacides selon la présente invention sont des oligomères ou des homopolymères comprenant des unités récurrentes acide glutamique ou aspartique ou des copolymères comprenant un mélange de ces deux types d'unités "acide aminé". Les unités considérées dans ces polymères sont des acides aminés ayant la configuration D ou 5 L ou D/L et sont liées par leurs positions alpha ou gamma pour l'unité glutamate ou acide glutamique et alpha ou bêta pour l'unité acide aspartique ou aspartate.

Les unités "acide aminé" préférées de la chaîne polyaminoacide principale sont celles ayant la configuration L et une liaison de type alpha.

Suivant un mode encore plus préféré de réalisation de l'invention, le polymère PO est un polyaminoacide formé par des unités acide aspartique et/ou des unités acide glutamiques, au moins une partie de ces unités étant porteuses de greffons comportant au moins un groupement hydrophobe GH. Ces polyaminoacides sont notamment du type de ceux décrits dans la demande de brevet PCT WO-A-00/30618.

Selon une première possibilité, le (ou les) PO de la formulation sont définis par la formule générale (**I**) suivante : 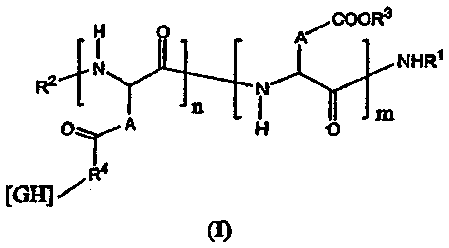 dans laquelle :
■ R¹ représente un H, un alkyle linéaire en C2 à C10 ou ramifié en C3 à C10, benzyle, une unité acide aminé terminale ou -R⁴-[GH] ;
■ R² représente un H, un groupe acyle linéaire en C2 à C10 ou ramifié en C3 à C10, un pyroglutamate ou -R⁴-[GH] ;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium,
   - les cations organiques avantageusement choisis dans le sous-groupe comprenant :
      - les cations à base d'amine,
      - les cations à base d'oligoamine,
      - les cations à base de polyamine (la polyethylèneimine étant particulièrement préférée),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine ;
■ R⁴ représente une liaison directe ou un "espaceur" à base de 1 à 4 unités acide aminé ;
■ A représente indépendamment un radical -CH₂- (unité acide aspartique) ou -CH₂-CH₂- (unité acide glutamique) ;
■ n/(n+m) est défini comme le taux de greffage molaire et sa valeur est suffisamment basse pour que PO mis en solution dans l'eau à pH 7 et à 25 °C, forme une suspension colloïdale de particules submicroniques de PO, de préférence n/(n + m) est compris entre 1 à 25 % molaire et mieux encore entre 1 et 15 % molaire ;
■ n + m est défini comme le degré de polymérisation et varie de 10 à 1000, de préférence entre 50 et 300 ;
■ GH représente un groupement hydrophobe.

Selon une deuxième possibilité, le (ou les) PO de la formulation répond à l'une des formules générales (**III**), (**III**) et (**IV**) suivantes: 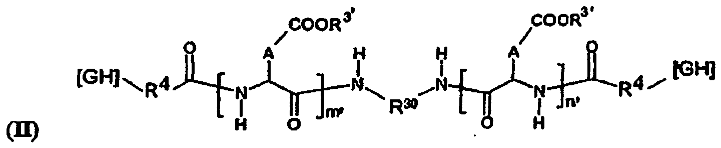 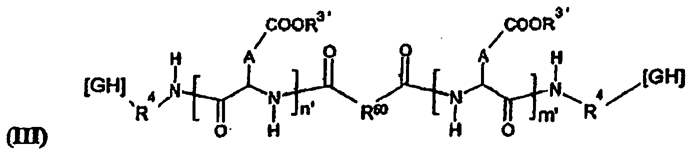 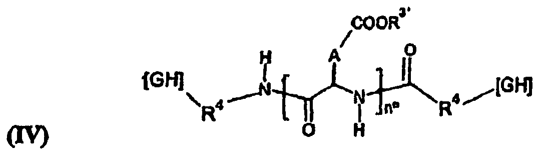 dans lesquelles :
■ GH représente un groupement hydrophobe ;
■ R³⁰ est un groupement alkyle linéaire en C2 à C6 ;
■ R^{3'} est un H ou une entité cationique, de préférence sélectionnée dans le groupe comprenant :
   - les cations métalliques avantageusement choisis dans le sous-groupe comprenant : le sodium, le potassium, le calcium, le magnésium,
   - les cations organiques avantageusement choisis dans le sous-groupe comprenant :
      - les cations à base d'amine,
      - les cations à base d'oligoamine,
      - les cations à base de polyamine (la polyéthylèneimine étant particulièrement préférée),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine,
■ R⁵⁰ est un groupement alkyle, dialcoxy ou diamine en C2 à C6;
■ R⁴ réprésente une liaison directe ou un "espaceur" à base de 1 à 4 unités acide aminé ;
■ A représente indépendamment un radical -CH₂- (unité acide aspartique) ou -CH₂-CH₂- (unité acide glutamique) ;
■ n' + m' ou n"est défini comme le degré de polymérisation et varie de 10 à 1000, de préférence entre 50 et 300.

Avantageusement, les n groupements GH du PO représentent chacun indépendamment les uns des autres un radical monovalent de formule suivante : 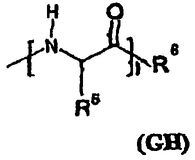 dans laquelle :
- R⁵ représente un méthyle(alanine), isopropyle (valine), isobutyle (leucine), secbutyle (isoleucine), benzyle (phénylalanine) ;
- R⁶ représente un radical hydrophobe comportant de 6 à 30 atomes de carbone;
- 1 varie de 0 à 6.

Selon une caractéristique remarquable, de l'invention, tout ou partie des groupements hydrophobes R⁶ des PO sont choisis de façon indépendante, dans le groupe de radicaux comportant :
■ un alcoxy linéaire ou ramifié comportant de 6 à 30 atomes de carbone et pouvant comporter au moins un hétéroatome (de préférence O et/ou N et/ou S) et/ou au moins une insaturation,
■ un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
■ un alcoxyaryle ou un aryloxyalkyle de 7 à 30 atomes de carbone et pouvant comporter au moins une insaturation et/ou au moins un héteroatome (de préférence O et/ou N et/ou S).

En pratique et sans que cela ne soit limitatif, le radical hydrophobe R⁶ du greffon du PO est issu d'un précurseur alcoolique choisi dans le groupe comprenant: l'octanol, le dodécanol, le tétradécanol, l'hexadécanol, l'octadécanol, l'oleylalcool, le tocophérol ou le cholestérol.

Selon une première forme de réalisation de l'invention, les chaînes principales des polyaminoacides sont des homopolymères d'alpha-L-glutamate ou d'acide alpha-L-glutamique.

Selon une deuxième forme de réalisation de l'invention, les chaînes principales des polyaminoacides sont des homopolymères d'alpha-L-aspartate ou d'acide alpha-L-aspartique.

Selon une troisième forme de réalisation de l'invention, les chaînes principales des polyaminoacides sont des copolymères d'alpha-L-aspartate/alpha-L-glutamate ou d'acide alpha-L-aspartique/alpha-L-glutamique.

Avantageusement, la distribution des unités acide aspartique et/ou acide glutatamique de la chaîne polyaminoacide principale du PO est telle que le polymère ainsi constitué est soit aléatoire, soit de type bloc, soit de type multibloc.

De préférence, le PO mis en oeuvre dans la formulation selon l'invention a une masse molaire qui se situe entre 2 000 et 100 000 g/mole, et de préférence entre 5 000 et 40 000 g/mole.

Suivant un premier mode préféré de réalisation de la formulation, le radical hydrophobe R⁶ du greffon du PO est issu d'un précurseur alcoolique formé par le tocophérol :
◆ 1 % ≤ [n/(n+m)]x 100 ≤ 10 %
◆ de préférence 3,5 % ≤ [n / (n+m)]x 100 ≤ 7,5 %
◆ n + m varie de 100 à 400, de préférence entre 120 et 300.

Suivant un deuxième mode préféré de réalisation de la formulation, le radical hydrophobe R⁶ du greffon du PO est issu d'un précurseur alcoolique formé par le cholestérol:
◆ 1 % ≤ [n/(n+m)]x 100 ≤ 10 %
◆ de préférence 3,5 % ≤ [n / (n+m)] x 100 ≤ 6,5 %
◆ n + m varie de 100 à 400, de préférence entre 120 et 300.

Dans ces deux modes préférés de réalisation de la formulation de l'invention, il est avantageux que la concentration en polymère [PO] soit comprise entre 15 et 50 mg/ml.

Selon une variante, le PO de la formulation selon l'invention est porteur d'au moins un greffon de type polyalkylène-glycol lié à une unité glutamate et/ou aspartate.

Avantageusement, ce greffon de type polyalkylène-glycol est de formule (V) suivante : 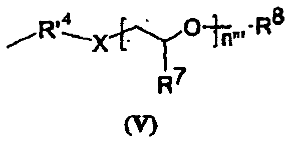 dans laquelle :
- R'⁴ représente une liaison directe ou un "espaceur" à base de 1 à 4 unités acide aminé ;
- X est un hétéroatome choisi dans le groupe comportant l'oxygène, l'azote ou un soufre ;
- R⁷ et R⁸ représentent indépendamment un H, un alkyle linéaire en C1 à C4;
- n"' varie de 10 à 1000, de préférence de 50 à 300.

En pratique, le polyalkylèneglycol est par exemple un polyethylène glycol.

Il est souhaitable, conformément à l'invention, que le pourcentage molaire de greffage du polyalkylène glycol varie de 1 à 30 %.

Les polyaminoacides PO sont en outre extrêmement intéressants, du fait qu'à un taux de greffage ajustable, ils se dispersent dans l'eau à pH 7,4 (par exemple avec un tampon phosphate) pour donner des suspensions colloïdales.

De plus, les principes actifs que sont les interférons ou d'autres PA choisis parmi les protéines, les peptides ou les petites molécules, peuvent s'associer spontanément à des nanoparticules comprenant ces polyaminoacides PO.

Il convient de comprendre que les PO à base de polyaminoacides contiennent des fonctions carboxyliques qui sont, soit neutres (forme COOH), soit ionisées (anion COO⁻), selon le pH et la composition. Pour cette raison, la solubilité dans une phase aqueuse est directement fonction du taux de COOH libres des PO (non greffé par le motif hydrophobe) et du pH. En solution aqueuse, le contre-cation peut être un cation métallique tel que le sodium, le calcium ou le magnésium, ou un cation organique tel que la triéthanolamine, la tris(hydroxyméthyl)-aminométhane ou une polyamine tel que la polyéthylèneimine.

Les PO de type polyaminoacides susceptibles d'être utilisés dans la formulation de l'invention sont, par exemple, obtenus par des méthodes connues de l'homme de l'art. Les polyaminoacides statistiques peuvent être obtenus par greffage du greffon hydrophobe, préalablement fonctionnalisé par "l'espaceur", directement sur le polymère par une réaction classique de couplage. Les PO polyaminoacides blocs ou multiblocs peuvent être obtenus par polymérisation séquentielle des anhydrides de N-carboxy-aminoacides (NCA) correspondants.

On prépare par exemple un polyaminoacide, homopolyglutamate, homopolyaspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-aminoacides (NCA), décrite, par exemple, dans l'article "Biopolymers, 1976, 15, 1869 et dans l'ouvrage de H.R. Kricheldorf "alpha-Aminoacid-N-carboxy Anhydrides and related Heterocycles" Springer Verlag (1987). Les dérivés d'NCA sont de préférence des dérivés NCA-O-Me, NCA-O-Et ou NCA-O-Bz (Me = Méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type d'acide poly(alpha-L-aspartique), acide poly(alpha-L-glutamique), acide poly(alpha-D-glutamique) et acide poly(gamma-L-glutamique) de masses variables sont disponibles commercialement. L'acide polyaspartique de type alpha-bêta est obtenu par condensation de l'acide aspartique (pour obtenir un polysuccinimide) suivie d'une hydrolyse basique (cf. Tomida et al. Polymer 1997,38,4733-36).

Le couplage du greffon avec une fonction acide du polymère est réalisé aisément par réaction du polyaminoacide en présence d'un carbodiimide comme agent de couplage et optionnellement, un catalyseur tel que la 4-diméthylaminopyridine et dans un solvant approprié tel que le diméthylformamide (DMF), la N-méthyl pyrrolidone (NMP) ou le diméthylsulfoxide (DMSO). Le carbodiimide est par exemple, le dicyclohoxylcarbodiimide ou le diisopropylcarbodiimide. Le taux de greffage est contrôlé chimiquement par la stoechiométrie des constituants et réactifs ou le temps de réaction. Les greffons hydrophobes fonctionnalisés par un "espaceur" sont obtenus par couplage peptidique classique ou par condensation directe par catalyse acide. Ces techniques sont bien connues de l'homme de l'art.

Pour la synthèse de copolymère bloc ou multibloc, on utilise des dérivés NCA préalablement synthétisés avec le greffon hydrophobe. Par exemple, le dérivé NCA-hydrophobe est copolyméris6 avec le NCA-O-Heuzyl puis on enlève par hydrolyse sélectivement les groupements benzyliques.

La synthèse de polyaminoacides PO conduit préférablement à des suspensions aqueuses de nanoparticules de PO.

De telles suspensions peuvent être transformées en poudres de nanoparticules de PO par séchage, de manière appropriée et connue de l'homme de l'art, comme par exemple : chauffage (étuve....), mise sous vide, utilisation de dessiccants, lyophilisation, atomisation.

Ces nanoparticules de PO, en suspension ou à l'état pulvérulent, forment une matière première pour la préparation des formulations selon l'invention.

A ce propos, il peut être précisé que les formulations selon l'invention résultent de l'association non covalente de nanoparticules à base d'au moins un PO et d'au moins un PA, dans un milieu liquide aqueux.

Pour la préparation, le PO et/ou l'iaterleukine(s) (et/ou l'éventuel PA supplémentaire) peut être sous forme solide (de préférence poudre) et/ou sous forme liquide (de préférence suspension aqueuse colloïdale).

L'association interIeukine(s)/PO signifie au sens du présent exposé que le (ou les) interleukine(s) est (sont) associé(s) au(x) pblymère(s) PO [e.g. un ou plusieurs polyaminoacide(s)] par une ou plusieurs liaisons autre(s) qu'une (ou que des) liaison(s) chimique(s) covalente(s).

Les techniques d'association d'un ou de plusieurs interleukines aux PO selon l'invention, sont décrites notamment dans la demande de brevet WO-A-00/30618. Elles consistent à incorporer au moins une interleukine (et un ou plusieurs autres principe(s) actif(s) éventuels) dans le milieu liquide contenant des nanoparticules de PO, de manière à obtenir une suspension colloïdale de nanoparticules chargées en ou associées avec une ou plusieurs interleukines (et un ou plusieurs autres principe(s) actif(s) éventuels).

L'invention a donc également pour objet un procédé de préparation de la formulation susvisée.

Selon un premier mode préféré de mise en oeuvre, ce procédé est caractérisé en ce qu'il consiste essentiellement :
◆ à mettre en oeuvre une suspension colloïdale de nanoparticules d'au moins un PO,
◆ à mélanger cette suspension colloïdale de nanoparticules de PO avec au moins une interleukine (et un ou plusieurs autres principe(s) actif(s) éventuels), de préférence en solution aqueuse,
◆ à ajouter éventuellement au moins un excipaient
◆ au besoin à ajuster le pH et/ou l'osmolarité et,
◆ éventuellement à filtrer la suspension ainsi obtenue.

Avantageusement, l'interleukine(s) (et un ou plusieurs autres principe(s) actifs) éventuels) est sous forme de suspension ou de solution aqueuse pour le mélange avec la suspension colloïdale de nanoparticules de PO.

Selon un second mode de mise en oeuvre, ce procédé est caractérisé en ce qu'il consiste essentiellement :
◆ à mettre en oeuvre une poudre d'au moins un polymère PO,
◆ à mélanger cette poudre avec une suspension ou solution aqueuse d'au moins une interleukine (et un ou plusieurs autres principe(s) actifs) éventuels), de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

Les formulations ainsi obtenues peuvent également être mises sous formes de gels, de poudre ou de film par les méthodes classiques connues de l'homme de l'art telles que la concentration par diafiltration ou évaporation, le couchage, l'atomisation ou la lyophilisation, entre autres. Ces méthodes peuvent être éventuellement combinées.

D'où il s'ensuit un troisième mode de mise en oeuvre du procédé de préparation des formulations liquides selon l'invention, ce troisième mode consistant essentiellement :
◆ à mettre en oeuvre une poudre issue du séchage de la formulation liquide selon l'invention telle que définie ci-dessus,
◆ à mélanger cette poudre avec un milieu liquide aqueux, de préférence sous agitation,
◆ à ajouter éventuellement au moins un excipient,
◆ au besoin à ajuster le pH et/ou l'osmolarité et,
◆ éventuellement à filtrer la suspension ainsi obtenue.

Les excipients susceptibles d'être rajoutés sont par exemple des antimicrobiens, des tampons, des antioxydants, des agents permettant d'ajuster l'isotonicité qui sont connus de l'homme de l'art. On pourra par exemple se référer à l'ouvrage : *Injectable Drug Development,* P.K. Gupta et al., Intelpharm Press, Denver,Colomdo 1999.

La filtration éventuelle de la formulation liquide sur des filtres de porosité égale, par exemple, à 0,2 µm, permet de la stériliser. Elle peut être ainsi directement injectée à un patient.

Tous ces exemples de préparation de formulations liquides selon l'invention sont avantageusement réalisés en atmosphère et à température ambiantes (25 °C e.g.).

Suivant une disposition intéressante de la formulation selon l'invention, sa traction massique en interleukine(s) non associée(s) aux particules submicroniques [imterleuldne(s) non associée(s)] en % en poids est telle que :
○ [interleukine(s) non associée(s)] ≤ 1
○ de préférence [interleukine(s) non associée(s)] ≤ 0,5
○ et plus préférenciellement [interleukine(s) non associée(s)] ≤ 0,1. Conformément à l'invention, l'interleukine préférée est l'interleukine 2.

Selon un autre de ses aspects, invention englobe tout produit dérivé obtenu à partir de la formulation liquide selon invention telle que définie supra et comprenant des particules submicroniques, formées par des associations non covalentes PO / interleukine telles que définies ci-dessus.

En pratique, ces produits dérivés peuvent notamment être constitués par des poudres, des gels, des implants ou des films, entre autres.

En outre, l'invention vise tout précurseur de la formulation liquide injectable telle que définie supra.

S'agissant toujours de ces produits dérivés, il doit être souligné que l'invention concerne également un procédé de préparation d'une poudre dérivée de la formulation telle que définie supra, ce procédé étant caractérisé en ce que ladite poudre est obtenue par séchage de !a formulation telle que définie ci-desus.

La formulation selon l'invention est de préférence pharmaceutique, sans exclure les formulations cosmétiques, diététiques ou phytosanitaires comprenant au moins un PO tel que défini ci-dessus et au moins une interleukine et éventuellement au moins un autre principe actif.

Selon l'invention, l'éventuel principe actif supplémentaire autre qu'une interleukine, peut être une protéine, une glycoprotéine, une protéine liée à une ou plusieurs chaînes polyalkylèneglycol [de préférence PolyEthylèneGlycol (PECi) : "protéine-PEGylée"], un polysaccharide, un liposaccharide, un oligonucléotide, un polynucléotide ou un peptide. Ce principe actif supplémentaire peut être sélectionné parmi les hémoglobines, les cytochromes, les albumines, les interférons, les cytokines, les antigènes, les anticorps, l'érythropoïétine, l'insuline, les hormones de croissance, les facteurs VIII et IX, les facteurs stimulants de l'hématopoïèse ou leurs mélanges.

Selon une variante, ce principe actif supplémentaire est une "petite" molécule organique hydrophobe, hydrophile ou amphiphile, par exemple les peptides tels que la leuprolide ou la cyclosporine ou les petites molécules telles que celles appartenant à la famille des anthracyclines, des taxoïdes ou des camptothécines et leurs mélanges.

Parmi les qualités primordiales de la formulation selon l'invention, figurent son caractère injectable et sa capacité à former un dépôt sur le site d'infection, in vivo, par gélification ou encore par agrégation des nanoparticules, en présence de protéines physiologiques ou analogues.

La formulation selon l'invention peut notamment être injectée par voie parentérale, sous-cutanée, intramusculaire, intradermique, intrapéritonéale, intracérébrale ou dans une tumeur.

La formulation selon l'invention peut aussi être administrée par voie orale, nasale, vaginale, oculaire ou buccale.

Avantageusement, la formulation est destinée à la préparation de médicaments, en particulier pour administration parentérale, sous-cutanée, intramusculaire, intradermique, intrapéritonéale, intracérébrale ou dans une tumeur, voire par voie orale, nasale, vaginale ou oculaire.

Bien que la formulation selon l'invention soit de préférence pharmaceutique, cela n'exclut pas pour autant les formulations cosmétiques, diététiques ou phytosanitaires comprenant au moins un PO tel que défini ci-dessus et au moins un principe actif correspondant.

Selon encore un autre de ses aspects, invention vise un procédé de préparation de médicaments, en particulier pour administration parentérale, sous-cutanée, intramusculaire, intradermique, intrapéritonéale, intracérébrale ou dans une tumeur, voire par voie orale, nasale, vaginale où oculaire, caractérisé en ce qu'il consiste essentiellement à mettre en oeuvre au moins une formulation sus-définie et/ou tout produit dérivé et/ou tout précurseur de ladite formulation.

L'invention concerne également l'utilisation thérapeutique de la formulation telle que décrite dans le présent exposé, consistant essentiellement à administrer ladite formulation par voie parentérale, sous-cutanée, intramusculaire, intradermique, intrapéritonéale, intracérébrale ou dans une tumeur, voire par voie orale, nasale, vaginale ou oculaire de manière à ce qu'elle forme un dépôt gélifié/réticulé sur le site d'injection

L'invention sera mieux comprise et ses avantages et variantes de misa en oeuvre ressortiront bien des exemples qui suivent et qui décrivent la synthèse des PO formés par des polyaminoacides greffés par un groupement hydrophobe, leur transformation en système de libération prolongée d'une interleukine, à savoir en formulation selon l'invention (suspension colloïdale aqueuse stable) et qui démonstrent de la capacité d'un tel système non seulement de s'associer à une interleukine, mais surtout de gélifier/réticuler pour libérer de manière très prolongée *in vivo* les interleukines.

### DESCRIPTION DES FIGURES

Figure 1 : Courbes des Concentrations plasmatiques d'IL2 (picogramme/ml) relevées chez le singe après injection sous cutanée
   - de la formulation d'IL2 (E) selon l'invention (exemple 7) :
      → courbe -□-□-
   - de la annulation d'IL2 (F) témoin hors de l'invention (exemple 7) :
      → courbe -●-●-,
   - et de la formulation d'IL2 (G) témoin hors de invention (exemple 7) :
      → courbe -■-■-,
en fonction du temps T en heures et à une dose d'IL2 de 0,5 mg/kg.

### EXEMPLES

### Exemple 1 : Polymère amphiphile P1.

### Synthèse d'un polyglutamate greffé par l'alpha-tocophérol d'origine synthétique

On solubilise 5,5 g d'un alpha-L-polyglutamate (de masse équivalente à environ 10 000 par rapport à un standard en polyoxyéthylène et obtenue par polymérisation de NCAGluOMe suivi d'une hydrolyse comme décrits dans la demande de brevet FR A-2 80122t7 dans 92 ml de diméthylformamide (DMP) en chauffant à 40°C pendant 2 heures, Une fois le polymère solubilisé, on laisse revenir la température à 25 oc et on ajoute successivement 1,49 g de D,L-alpha-tocophérol (> 98 % obtenu de Fluka®) préalablement solubilisé dans 6 ml de DMF, 0,09 g de 4-dimethylaminopyridine préalablement solubilisé dans 6 ml de DMF et 0,57 g de diisopropylcarbodiimide préalablement solubilisé dans 6 ml de DMF. Après 8 heures à 25 °C sous agitation, le milieu réactionnel est versé dans 800 ml d'eau contenant 15 % de chlorure de sodium et d'acide chlorhydrique (pH 2). Le polymère précipité est ensuite récupéré par filtration, lavé par de l'acide chlorhydrique 0,1 N puis par de l'eau. Le polymère est ensuite resolubilisé dans 75 ml de DMF puis reprécipité dans de l'eau contenant comme précédemment du sel et de l'acide à pH 2. Après deux lavages à l'eau, on lave plusieurs fois par de l'éther diisopropylique. Le polymère est ensuite séché à l'étuve sous vide à 40 °C. On obtient un rendement de l'ordre de 85 %.

### Exemple 2 : Polymères ampbiphiles P1, P2, P3, P4, P5 et P6.

Ces polymères sont obtenus de la même façon que pour l'obtention du polymère P1. Le tableau 1 ci-dessous résume les caractéristiques de ces polymères. Celles du polymère P1 sont données à titre de comparaison.

**TABLEAU 1**

| Polymère | Mn¹ g/mol du polyglutamate | groupement hydrophobe | % de greffage (RMN)² | Mn¹ g/mol du polymère |
|---|---|---|---|---|
| P1 | 10 000 | alpha-tocophérol³ | 7 | 13 900 |
| P2 | 10 000 | alnha-tocophérol³ | 4 | 14 400 |
| P3 | 16 900 | alpha-tocophérol³ | 4 | 15 200 |
| P4 | 10 000 | cholestérol | 5 | 11 500 |
| P5 | 16 900 | cholestérol | 5 | 12 900 |
| P6 | 10000 | n-Dodécanol | 15 | 11 500 |

| | | | | |
|---|---|---|---|---|
| ¹ En équivalent polyoxyéthylène. ² Taux de greffage molaire estimé par la RMN du proton. ³ d'origine synthétique | | | | |

### Exemple 3: Préparation d'une formulation d'InterLeuKine-2 (IL2) longue action selon la présente invention à base du polymère P3.

On introduit dans un flacon la quantité de poudre lyophilisée du polymère amphiphile et d'eau stérile nécessaire pour obtenir une concentration en polymère égale à X =1,3 fois la concentration finale recherchée dans la formulation. La dissolution du polymère est prolongée 16 heures sous agitation magnétique.

La quantité nécessaire d'IL2 lyophilisée (Prospec) est concentrée à X/(X-1) fois la concentration finale recherchée.

La concentration précise de la solution d' IL2 concentrée est déterminée par dosage UV à 280 nm à l'aide d'un spectrophotomètre UV Perkin Elmer Lambda 35. Cette solution d'IL2 est filtrée sur 0,8-0,2 µm et stockée à 4°C. Son pH est ajusté à pH 11 par ajout de NaOH 1 M. On nomme Y le rapport de la concentration en protéine de cette solution à la concentration souhaitée dans la formulation.

On procède ensuite au mélange à température ambiante de la solution de protéine et de la solution de polymère. Pour un volume de polymère, on rajoute X-1 volumes de solution de protéine. Le pH et l'osmolarité sont ajustés à pH 7,4 ± 0,2 et à 300 ± 20 mOsm.

Ainsi, pour préparer une formulation d'IL2 longue action selon l'invention à base du polymère P3 contenant 20 mg/ml de polymère P3 et 2,5 mg/ml d'IL2, la solution initiale de polymère est concentrée à 26 mg/ml. La solution initiale d'IL2 est concentrée à 11 mg/ml. Pour un volume de polymère, on rajoute 0,3 volumes de solution de protéine.

### Exemple 4 : Mesure du diamètre hydrodynamique moyen des nanoparticules de différents polymères PO selon invention

Le diamètre hydrodynamique moyen des particules de polymère PO selon l'invention est mesuré selon le Mode opératoire Md défini ci-après.

Les solutions de PO sont préparées à des concentrations de 1 ou 2 mg/ml en milieu NaCl 0,15M et laissées sous agitation pendant 24 h. Ces solutions sont ensuite filtrées sur 0,8-0,2 µm, avant de les analyser en diffusion dynamique de la lumière grâce à un appareil de type Brookhaven, fonctionnant avec un faisceau laser de longueur d'onde 488 nm et polarisé verticalement. Le diamètre hydrodynamique des nanoparticules de polymère PO est calculé à partir de la fonction d'autocorrélation du champ électrique par là méthode des cumulants, comme décrit dans l'ouvrage « Surfactant Science Series » volume 22, Surfactant Solutions, Bd. R Zana, chap. 3, M. Dekker, 1984.

On obtient les résultats suivants pour les polymères PO P2 P3 P4 et P6 de l'exemple 2 :

**TABLEAU 2**

| Polymère | Diamètre hydrodynamique moyen (nm) |
|---|---|
| P2 | 60 |
| P3 | 90 |
| P4 | 30 |
| P6 | 15 |

### Exemple S : Association spontanée d'une protéine aux nanoparticules de polymère PO

Une solution de tampon phosphate à 25 mM est préparée à partir de poudre de NaH₂PO₄ (Sigma ref S-0751) et ajustée avec de la soude 1N (SDS ref 3470015) à pH = 7,2. Une suspension colloïdale de nanoparticules de polymère P1 est préparée par dissolution pendant une nuit du polymère lyophilisé à 5 mg/ml dans la solution de tampon phosphate précédente.

Une solution mère de BSA (Sigma A-2934) est préparée par dissolution pendant deux heures de protéine à 10 mg/ml dans le même tampon.

Les solutions mères ainsi que le tampon sont filtrées sur 0,22 µm. Des mélanges sont réalisés par ajout de volumes prédéterminée des deux solutions mères et dilution dans le tampon phosphate de façon a avoir au final une gamme d'échantillons ayant une concentration constante en polymère (0,1 mg/ml) et des concentrations croissantes de protéines (0 à 1,8 mg/ml).

Les échantillons sont laissés 5 heures à associer à 25 °C puis ils sont analysés par électrophorèse capillaire dans une méthode dite frontale ou il est possible de visualiser séparément la protéine et le complexe protéine - polymère. Pour plus de détails sur cette technique, on consultera l'article suivant : Gao JY, Dublin PL, Muhoberac BB, Anal. Chem. 1997, 69, 2945. Les analyses sont réalisées sur un appareil Agilent G16000A muni d'un capillaire à bulle en silice fondue (type G1600-62-232). La hauteur du premier plateau correspondant à la protéine libre permet de déterminer la concentration en BSA non associée. L'expérience montre que pour des quantités de protéines inférieures à 0,1 g de protéine par g de polymère, la protéine est associée aux nanoparticles de polymère.

### Exemple 6 : Détermination de la concentration de gélification C1 pour les polymères PO P1, P3 et P6.

Le test GI est appliqué à des formulations d'IL2 associées aux polymères P1, P3 et P6 des exemples 1 et 2. Les concentrations en protéines de ces formulations sont reportées dans le tableau ci dessous. La mesure du temps de relaxation des formulations en présence de BSA (concentration 30 mg/ml) s'effectue selon le mode opératoire du test GL La concentration critique C1, pour laquelle le temps de relaxation excède 1s est reportée sur le tableau 3 pour l'IL-2 :

**TABLEAU 3 :**

| | | | |
|---|---|---|---|
| Concentration de gélification induite pour des formulations d'IL2 | | | |

| Polymère | P1 | P3 | P6 |
|---|---|---|---|
| Concentration en IL2 (mg/ml) | 2,5 | 2,5 | 2,5 |
| Concentration C1 (mg/ml) | 17 | 17 | >50 |

### Exemple 7 : Pharmacocinétique de l'interleukine 2 (IL2) chez le singe après injection sous cutanée de diverses formulations à base de polyaminoacides amphiphiles.

Les formulations suivantes sont préparées selon le mode opératoire décrit dans l'exemple 3.

**TABLEAU 4**

| Révérence | Polymère | Concentration en polymère (mg/ml) | Concentration en IL2 (mg/ml) |
|---|---|---|---|
| E | P1 | 30 | 2,5 |
| F | P3 | 20 | 2,5 |
| G | P6 | 40 | 2,5 |

Les formulations E et F, qui ont une concentration en polymère supérieure à la concentration de gélification C1 mesurée dans l'exemple 6 appartiennent donc à la sélection selon l'invention. En revanche, la formulation G a une concentrations inférieure à la concentration de gélification C1 et n'appartient pas à la sélection salon l'invention.

Ces formulations sont injectées à la dose 0,5 mg / kg à des singes Cynomolgus. Des prélèvements de plasma sont effectués aux temps 1, 5, 11, 24, 36, 48, 72, 96, 120, 144, 168 et 240 heures. La concentration plasmatique en IL2 est mesurée sur ces prélèvements par dosage ELISA (kit Immunotech IM 3583).

Les temps Tmax et T50 pour les formulations E, F et G sont reportés dans le tableau 5 ci-dessous.

**TABLEAU S**

| Référence formulation | Tmax (h) | T50 (h) |
|---|---|---|
| E | 32 | 34,5 |
| F | 32 | 37,5 |
| G | 4 | 10,5 |

Ainsi, les formulations E et F, qui appartiennent à la sélection selon l'invention, présentent une durée de libération considérablement accrue par rapport à la formulation G qui n'appartient pas à la sélection selon l'invention.

### Exemple 8 : Observation de la gélification in vivo des formulations selon l'invention après injection sous-cutanée.

Le comportement sous cutané des formulations selon l'invention a été étudié chez le porc domestique. On a procédé à des injections sous la peau du ventre, à 4 mm de profondeur, de six porcs domestiques, avec 0,3 ml des formulations suivantes : Formulation A : solution aqueuse isotonique à pH 7,3 du polymère P6 de l'exemple 2 concentré à 45 mg/ml.

Formulation B : solution aqueuse isotonique à pH 7,3 du polymère P1 de l'exemple 1 concentré à 20 mg/ml.

Les sites injectés ont été prélevés 72 heures après administration. L'examen histologique révèle la présence d'un dépôt gélifié de polymère pour la formulation B. I se présente sous forme de plages uniformément colorées. Ce phénomène n'est en revanche pas observé pour la formulation A pour laquelle le polymère est infiltré entre les fibres de collagène.

On peut souligner que la matrice de polymère B est parfaitement bio dégradable car le tissu est complètement revenu à son état normal après 21 jours.

## Revendications

1. Formulation pharmaceutique liquide pour la libération prolongée d'interleukine(s), cette formulation comprenant au moins une interleukine, éventuellement au moins un autre principe actif (PA), de préférence en solution aqueuse, et une suspension colloïdale, aqueuse, de basse viscosité, à base de particules submicroniques de polymère (PO) biodégradable, hydrosoluble sélectionné dans le groupe consistant en les polyaminoacides, les polysaccharides, les gélatines ou leurs mélanges et porteur de groupements hydrophobes (GH), lesdites particules étant associées de façon non covalente avec ladite interleukine et éventuellement ledit autre principe actif (PA),
caractérisée :
◆ en ce que le milieu dispersif de la suspension est essentiellement constitué par de l'eau,
◆ en ce que sa concentration en [PO] est fixée à une valeur [PO] ≥ 0,9.C1 de sorte que ladite formulation pharmaceutique est apte à être injectée par voie parentérale et à former ensuite in vivo un dépôt gélifié, C1 représentant la concentration de "gélification induite" des particules de PO telle que mesurée dans un test GI comme décrit dans la description, cette formation de dépôt gélifié :
○ étant, d'une part, au moins en partie provoquée par au moins une protéine physiologique présente in vivo,
○ et permettant, d'autre part, de prolonger et de contrôler la durée de libération de l'interleukine et éventuellement du PA in vivo, au-delà de 24 h après l'administration,
◆ en ce qu'elle est liquide dans les conditions d'injection,
◆ et en ce qu'elle est également liquide à la température et/ou au pH physiologiques, et/ou en présence :
* d'électrolyte physiologique en concentration physiologique,
* et/ou d'au moins un tensioactif.

2. Formulation pharmaceutique liquide pour la libération prolongée d'interleukine(s) et, éventuellement d'autre(s) principe(s) actif(s) -PA-, cette formulation:
○ étant liquide en atmosphère ambiante,
○ étant également liquide à la température et/ou au pH physiologiques et/ou en présence :
* d'électrolyte physiologique en concentration physiologique,
* et/ou d'au moins un tensioactif,
○ et comprenant au moins une interleukine, éventuellement au moins un autre principe actif (PA), de préférence en solution aqueuse, et une suspension colloïdale, aqueuse, de basse viscosité, à base de particules submicroniques de polymère PO biodégradable, hydrosoluble sélectionné dans le groupe consistant en les polyaminoacides, les polysaccharides, les gélatines ou leurs mélanges et porteur de groupements hydrophobes (GH), lesdites particules étant associées de façon non covalente avec ladite interleukine et éventuellement ledit principe actif PA et le milieu dispersif de la suspension étant essentiellement constitué par de l'eau,
**caractérisée en ce que** sa concentration en [PO] est fixée à une valeur suffisamment élevée pour permettre la formation de dépôt gélifié in vitro, en présence d'au moins une protéine, ladite concentration en [PO] étant telle que [PO] ≥ 0,9.C1, C1 représentant la concentration de "gélification induite" des particules de PO telle que mesurée dans un test GI comme décrit dans la description.

3. Formulation selon l'une quelconque des revendications précédentes, **caractérisée en ce que** sa concentration en [PO] est telle que:
■ 20.C1 ≥ [PO] ≥ C1,
■ et mieux encore 10.C1 ≥ [PO] ≥ C1

4. Formulation selon l'une quelconque des revendications précédentes, **caractérisée en ce que** les polymères modifiés hydrophobes PO sont sélectionnés dans le groupe consistant en les polysaccharides choisis dans le groupe des pullulanes, des chitosans et des mucopolysaccharides.

5. Formulation selon l'une quelconque des revendications précédentes, **caractérisée en ce que** les groupements hydrophobes (GH) se situent latéralement à la chaîne.

6. Formulation selon l'une quelconque des revendications précédentes, **caractérisée en ce que** le polymère PO est un polyaminoacide formé par des unités acide aspartique et/ou des unités acide glutamique, au moins une partie de ces unités étant porteuses de greffons comportant au moins un groupement hydrophobe (GH).

7. Formulation selon la revendication 6, **caractérisée en ce que** le (ou les) PO est (ou sont) définis par la formule générale (1) suivante : 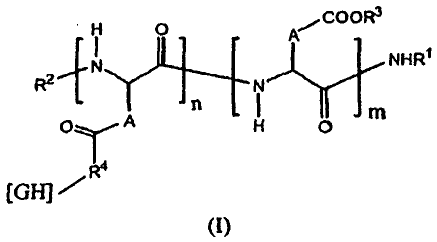 dans laquelle :
■ R¹ représente un H, un alkyle linéaire en C2 à C10 ou ramifié en C3 à C10, benzyle, une unité acide aminé terminale ou -R⁴-[GH] ;
■ R² représente un H, un groupe acyle linéaire en C2 à C10 ou ramifié en C3 à C10, un pyroglutamate ou -R⁴-[GH] ;
■ R³ est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine,
• les cations à base de polyamine (la polyethylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine ;
■ R⁴ représente une liaison directe ou un "espaceur" à base de 1 à 4 unités acide aminé ;
■ A représente indépendamment un radical -CH₂- (unité acide aspartique) ou -CH₂-CH₂- (unité acide glutamique) ;
■ n/(n+m) est défini comme le taux de greffage molaire et varie de 0,5 à 100 % molaire;
■ n/(n+m) est défini comme le taux de greffage molaire et sa valeur est suffisamment basse pour que PO mis en solution dans l'eau à pH 7 et à 25 °C, forme une suspension colloïdale de particules submicroniques de PO, de préférence n/(n + m) est compris entre 1 à 25 % molaire et mieux encore entre 1 et 15 % molaire;
■ n + m varie de 10 à 1000, de préférence entre 50 et 300 ;
■ GH représente un groupement hydrophobe.

8. Formulation selon la revendication 6, **caractérisée en ce que** le (ou les) PO répond(ent) à l'une des formules générales (II), (III) et (IV) suivantes : 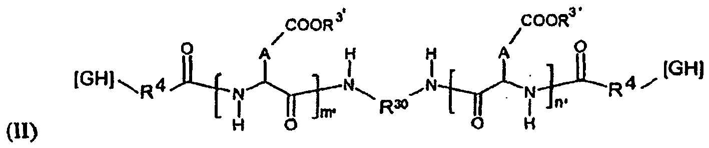 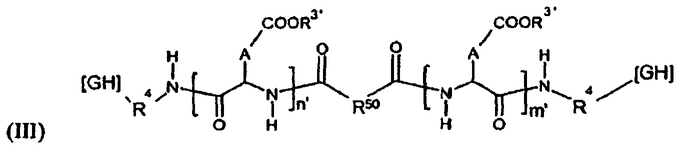 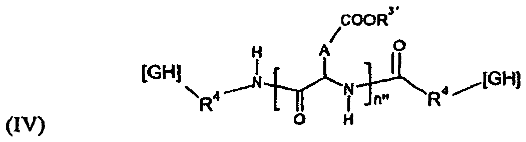 dans lesquelles :
■ GH représente un groupement hydrophobe ;
■ R³⁰ est un groupement alkyle linéaire en C2 à C6 ;
■ R^{3'} est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine,
• les cations à base de polyamine (la polyéthylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine,
■ R⁵⁰ est un groupement alkyle, dialcoxy ou diamine en C2 à C6 ;
■ R⁴ représente une liaison directe ou un "espaceur" à base de 1 à 4 unités acide aminé ;
■ A représente indépendamment un radical -CH₂- (unité acide aspartique) ou -CH₂-CH₂- (unité acide glutamique);
■ n' + m' ou n" est défini comme le degré de polymérisation et varie de 10 à 1000, de préférence entre 50 et 300.

9. Formulation selon la revendication 7 ou 8, **caractérisée en ce que** les n groupements GH du PO représentent chacun indépendamment les uns des autres un radical monovalent de formule suivante : 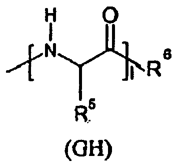 dans laquelle :
- R⁵ représente un méthyle (alanine), isopropyle (valine), isobutyle (leucine), secbutyle (isoleucine), benzyle (phénylalanine) ;
- R⁶ représente un radical hydrophobe comportant de 6 à 30 atomes de carbone ;
- I varie de 0 à 6.

10. Formulation selon la revendication 9, **caractérisée en ce que** tout ou partie des radicaux hydrophobes R⁶ des PO sont choisis de façon indépendante dans le groupe de radicaux comportant :
■ un alcoxy linéaire ou ramifié comportant de 6 à 30 atomes de carbone et pouvant comporter au moins un hétéroatome (de préférence O et/ou N et/ou S) et/ou au moins une insaturation,
■ un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
■ un alcoxyaryle ou un aryloxyalkyle de 7 à 30 atomes de carbone et pouvant comporter au moins une insaturation et/ou au moins un hétéroatome (de préférence O et/ou N et/ou S).

11. Formulation selon la revendication 9 ou 10, **caractérisée en ce que** le radical hydrophobe R⁶ du greffon du PO est issu d'un précurseur alcoolique choisi dans le groupe comprenant: l'octanol, le dodécanol, le tétradécanol, l'hexadécanol, l'octadécanol, l'oleylalcool, le tocophérol ou le cholestérol.

12. Formulation selon la revendication 6, **caractérisée en ce que** le PO est constitué d'un homopolymère d'alpha-L-glutamate ou d'acide alpha-L-glutamique.

13. Formulation selon la revendication 6, **caractérisée en ce que** le PO est constitué d'un homopolymère d'alpha-L-aspartate ou d'acide alpha-L-aspartique.

14. Formulation selon la revendication 6, **caractérisée en ce que** le PO est constitué d'un copolymère d'alpha-L-aspartate/alpha-L-glutamate ou d'acide alpha-L-aspartique/alpha-L-glutamique.

15. Formulation selon la revendication 14, **caractérisée en ce que** dans le PO, la distribution des unités acide aspartique et/ou acide glutamique porteuses de greffons comportant au moins un motif GH est telle que le polymère ainsi constitué est soit aléatoire, soit de type bloc, soit de type multibloc.

16. Formulation selon la revendication 1, **caractérisée en ce que** la masse molaire du PO se situe entre 2 000 et 100 000 g/mole, et de préférence entre 5 000 et 40 000 g/mole.

17. Formulation selon les revendications 7 et 9, **caractérisée en ce que** le radical hydrophobe R⁶ du greffon du PO est issu d'un précurseur alcoolique formé par le tocophérol et **en ce que** :
◆ 1 % ≤ [n / (n+m)]x 100 ≤ 10 %
◆ de préférence 3,5 % ≤ [n / (n+m)]x 100 ≤ 7,5 %
◆ n + m varie de 100 à 400, de préférence entre 120 et 300.

18. Formulation selon les revendications 7 et 9, **caractérisée en ce que** le radical hydrophobe R⁶ du greffon du PO est issu d'un précurseur alcoolique formé par le cholestérol:
◆ 1 % ≤ [n / (n+m)]x 100 ≤ 10 %
◆ de préférence 3,5 % ≤ [n / (n+m)] x 100 ≤ 6,5 %
◆ n + m varie de 100 à 400, de préférence entre 120 et 300.

19. Formulation selon la revendication 17 ou 18, **caractérisée en ce que** la concentration en polymère [PO] est comprise entre 15 et 50 mg/ml.

20. Formulation selon l'une quelconque des revendications 1 à 19, **caractérisée en ce que** sa viscosité à 20 °C est inférieure ou égale à 5 Pa.s

21. Formulation selon l'une quelconque des revendications 1 à 20, **caractérisée en ce que** sa fraction massique en interleukine(s) non associée(s) aux particules submicroniques [interteukine(s) non associée(s)] en % en poids est telle que :
○ [interleukine(s) non associée(s)] ≤ 1
○ de préférence [interleukine(s) non associée(s)] ≤ 0,5
○ et plus préférentiellement [interleukine(s) non associée(s)] ≤ 0,1.

22. Formulation selon l'une quelconque des revendications 1 à 21, **caractérisée en ce que** l'interleukine est l'interleukine 2.

23. Formulation selon l'une quelconque des revendications 1 à 22, **caractérisée en ce que** le (ou les) principe(s) actif(s) supplémentaire(s) autre que l'(ou les)interleukine(s) est une protéine, une glycoprotéine, une protéine liée à une ou plusieurs chaînes polyalkylèneglycol [de préférence polyéthylèneglycol (PEG) : "protéine-PEGylée"], un polysaccharide, un liposaccharide, un oligonucléotide, un polynucléotide ou un peptide, ce (ou ces) principe(s) actif(s) supplémentaire(s) étant de préférence sélectionné(s) parmi les hémoglobines, les cytochromes, les albumines, les interférons, les cytokines, les antigènes, les anticorps, l'érythropoïétine, l'insuline, les hormones de croissance, les facteurs VIII et 1X, les facteurs stimulants de l'hématopoïèse ou leurs mélanges.

24. Formulation selon l'une quelconque des revendications 1 à 23, **caractérisée en ce qu'**elle est injectable par voie parentérale, sous-cutanée, intramusculaire, intradermique, intrapéritonéale, intracérébrale ou dans une tumeur.

25. Formulation selon l'une quelconque des revendications 1 à 24, **caractérisée en ce qu'**elle est destinée à la préparation de médicaments, en particulier pour administration parentérale, sous-cutanée, intramusculaire, intradermique, intrapéritonéale, intracérébrale ou dans une tumeur, voire par voie orale, nasale, vaginale ou oculaire.

26. Procédé de préparation de médicaments, en particulier pour administration parentérale, sous-cutanée, intramusculaire, intradermique, intrapéritonéale, intracérébrale ou dans une tumeur, voire par voie orale, nasale, vaginale ou oculaire, **caractérisé en ce qu'**il consiste essentiellement à mettre en oeuvre au moins une formulation selon l'une quelconque des revendications 1 à 25.

27. Produit dérivé **caractérisé en ce qu'**il comprend des particules submicroniques, formées par des associations non covalentes PO/PA telles que définies dans la revendication 1 et **en ce qu'**il est obtenu à partir de la formulation selon l'une quelconque des revendications 1 à 25.

28. Produit dérivé selon la revendication 27, **caractérisé en ce qu'**il est constitué par une poudre ou par un gel.

29. Procédé de préparation de la formulation selon l'une quelconque des revendications 1 à 25, **caractérisé en ce qu'**il consiste essentiellement :
◆ à mettre en oeuvre une suspension colloïdale de nanoparticules d'au moins un PO,
◆ à mélanger cette suspension colloïdale de nanoparticules de PO avec au moins une interleukine (et un ou plusieurs autres principe(s) actif(s) éventuels), de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

30. Procédé selon la revendication 29, **caractérisé en ce que** le (ou les) PA(s) est (sont) sous forme de suspension ou de solution aqueuse pour le mélange avec la suspension colloïdale de nanoparticules de PO.

31. Procédé de préparation de la formulation selon l'une quelconque des revendications 1 à 25, **caractérisé en ce qu'**il consiste essentiellement :
◆ à mettre en oeuvre une poudre d'au moins un polymère PO,
◆ à mélanger cette poudre avec une suspension ou solution aqueuse d'au moins une interleukine (et un ou plusieurs autres principe(s) actif(s) éventuels), de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

32. Procédé de préparation de la formulation selon l'une quelconque des revendications 1 à 25, **caractérisé en ce qu'**il consiste essentiellement :
◆ à mettre en oeuvre une poudre issue du séchage de la formulation liquide selon l'une quelconque des revendications 1 à 25,
◆ à mélanger cette poudre avec un milieu liquide aqueux, préférence sous agitation,
◆ à ajouter éventuellement au moins un excipient,
◆ au besoin à ajuster le pH et/ou l'osmolarité et,
◆ éventuellement à filtrer la suspension ainsi obtenue.

33. Procédé de préparation d'une poudre dérivée de la formulation selon l'une quelconque des revendications 1 à 25, **caractérisé en ce que** ladite poudre est obtenue par séchage de la formulation selon l'une quelconque des revendications 1 à 25.

## Claims

1. Liquid pharmaceutical formulation for the prolonged release of interleukin(s), this formulation comprising at least one interleukin, optionally at least one other active principle active principle (AP), preferably in aqueous solution and an aqueous colloidal suspension of low viscosity based on submicronic particles of water-soluble biodegradable polymer (PO) selected from the group comprising polyamino acids, polysaccharides, gelatins and mixtures thereof and carrying hydrophobic groups (HG), said particles being non-covalently associated with said interleukin and optionally with said other active principle active principle (AP), **characterized in that**:
◆ the dispersion medium of the suspension consists essentially of water,
◆ its concentration of [PO] is fixed to a value [PO] ≥ 0.9.C1, such that said pharmaceutical formulation is capable of being injected parenterally and then forming a gelled deposit in vivo, C1 being the "induced gelling" concentration of the particles of PO such as measured in an IG test such as described in the description, this formation of a gelled deposit:
o on the one hand being at least partly caused by at least one physiological protein present in vivo,
o and on the other hand making it possible to prolong and control the in vivo release time of the interleukin and optionally of the AP beyond 24 h after administration,
◆ it is liquid under the injection conditions,
◆ and it is also liquid at the physiological temperature and/or physiological pH and/or in the presence of:
* a physiological electrolyte in a physiological concentration,
* and/or at least one surfactant.

2. Liquid pharmaceutical formulation for the prolonged release of interleukin(s) and optionally other active principle(s) (AP), this formulation:
○ being liquid in the ambient atmosphere,
○ also being liquid at the physiological temperature and/or physiological pH and/or in the presence of:
* a physiological electrolyte in a physiological concentration,
* and/or at least one surfactant,
○ and comprising at least one interleukin, optionally at least one other active principle active principle (AP), preferably in aqueous solution and an aqueous colloidal suspension of low viscosity based on submicronic particles of water-soluble biodegradable polymer PO selected from the group comprising polyamino acids, polysaccharides, gelatins and mixtures thereof and carrying hydrophobic groups HG, said particles being non-covalently associated with said interleukin and optionally with said active principle AP, and the dispersion medium of the suspension consisting essentially of water,
**characterized in that** its concentration of [PO] is such that [PO] ≥ 0.9.C1, C1 being the "*induced gelling*" concentration of the particles of PO such as measured in an IG test such as described in the description.

3. Formulation according to any one of the preceding claims, **characterized in that** its concentration of [PO] is such that:
■ 20.C1 ≥ [PO] ≥ C1,
■ and particularly preferably 10.C1 ≥ [PO] ≥ C1.

4. Formulation according to any one of the preceding claims, **characterized in that** the hydrophobically modified polymers PO are selected from the group consisting in polysaccharides chosen from the group of pullulans, chitosans and mucopolysaccharides.

5. Formulation according to any one of the preceding claims, **characterized in that** the hydrophobic groups (HG) are attached laterally to the chain.

6. Formulation according to any one of the preceding claims, **characterized in that** the polymer PO is a polyamino acid formed of aspartic units and/or glutamic units, at least some of these units carrying grafts containing at least one hydrophobic group (HG).

7. Formulation according to claim 6, **characterized in that** the PO is (are) defined by general formula (I) below: 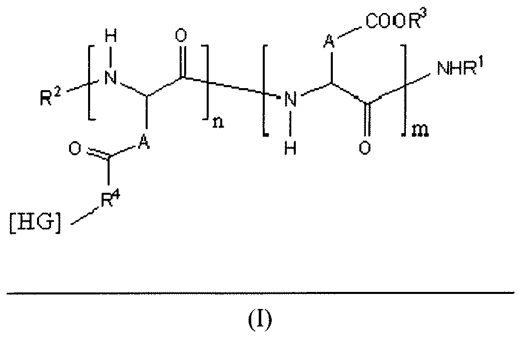 in which:
■ R¹ is H, a linear C2 to C10 alkyl or branched C3 to C10 alkyl, benzyl, a terminal amino acid unit or -R⁴-[HG];
■ R² is H, a linear C2 to C10 acyl or branched C3 to C 10 acyl group, a pyroglutamate or -R⁴-[HG];
■ R³ is H or a cationic entity preferably selected from the group comprising:
- metal cations advantageously selected from the subgroup comprising sodium, potassium, calcium and magnesium,
- organic cations advantageously selected from the subgroup comprising:
• cations based on amine,
• cations based on oligoamine,
• cations based on polyamine (polyethylenimine being particularly preferred),
• cations based on amino acid(s) advantageously selected from the class comprising cations based on lysine or arginine,
- or cationic polyamino acids advantageously selected from the subgroup comprising polylysine and oligolysine;
■ R⁴ is a direct bond or a "spacer" based on 1 to 4 amino acid units;
■ A independently is a radical -CH₂- (aspartic unit) or -CH₂-CH₂- (glutamic unit);
■ n/(n + m) is defined as the molar grafting rate and varies from 0.5 to 100 mol%;
■ n/(n + m) is defined as the molar grafting rate and its value is sufficiently low for PO, dissolved in water at pH 7 and at 25°C, to form a colloidal suspension of submicronic particles of PO, n/(n + m) preferably being between 1 and 25 mol% and particularly preferably between 1 and 15 mol%;
■ n + m varies from 10 to 1000 and preferably between 50 and 300;
■ HG is a hydrophobic group.

8. Formulation according to claim 6, **characterized in that** the PO has (have) one of general formulae (II), (III) and (IV) below: 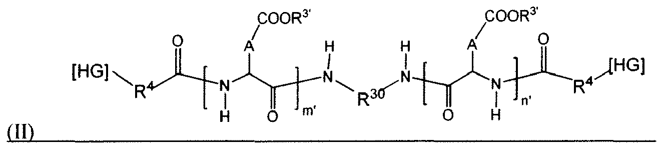 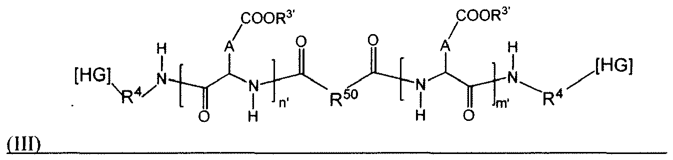 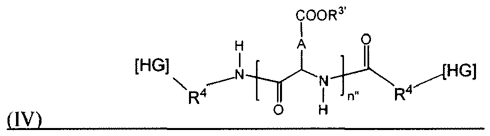 in which:
■ HG is a hydrophobic group;
■ R³⁰ is a linear C2 to C6 alkyl group;
■ R^{3'} is H or a cationic entity preferably selected from the group comprising:
- metal cations advantageously selected from the subgroup comprising sodium, potassium, calcium and magnesium,
- organic cations advantageously selected from the subgroup comprising:
• cations based on amine,
• cations based on oligoamine,
• cations based on polyamine (polyethylenimine being particularly preferred),
• cations based on amino acid(s) advantageously selected from the class comprising cations based on lysine or arginine,
- or cationic polyamino acids advantageously selected from the subgroup comprising polylysine and oligolysine;
■ R⁵⁰ is a C2 to C6 alkyl, dialkoxy or diamine group;
■ R⁴ is a direct bond or a "spacer" based on 1 to 4 amino acid units;
■ A independently is a radical -CH₂- (aspartic unit) or -CH₂-CH₂- (glutamic unit);
■ n' + m' or n" is defined as the degree of polymerization and varies from 10 to 1000 and preferably between 50 and 300.

9. Formulation according to claim 7 or 8, **characterized in that** the n HG of the PO each independently of one another are a monovalent radical of the formula below: 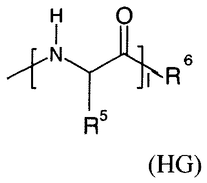 in which:
- R⁵ is a methyl (alanine), isopropyl (valine), isobutyl (leucine), sec-butyl (isoleucine) or benzyl (phenylalanine);
- R⁶ is a hydrophobic radical containing from 6 to 30 carbon atoms;
- 1 varies from 0 to 6.

10. Formulation according to claim 9, **characterized in that** all or some of the hydrophobic radicals R⁶ of the PO are independently selected from the group of radicals comprising:
■ a linear or branched alkoxy containing from 6 to 30 carbon atoms and optionally containing at least one heteroatom (preferably O and/or N and/or S) and/or at least one unit of unsaturation,
■ an alkoxy containing 6 to 30 carbon atoms, having one or more fused carbocyclic rings and optionally containing at least one unit of unsaturation and/or at least one heteroatom (preferably O and/or N and/or S),
■ an alkoxyaryl or an aryloxyalkyl having 7 to 30 carbon atoms and optionally containing at least one unit of unsaturation and/or at least one heteroatom (preferably O and/or N and/or S).

11. Formulation according to claim 9 or 10, **characterized in that** the hydrophobic radical R⁶ of the graft of the PO is derived from an alcohol precursor selected from the group comprising octanol, dodecanol, tetradecanol, hexadecanol, octadecanol, oleyl alcohol, tocopherol and cholesterol.

12. Formulation according to claim 6, **characterized in that** the PO consists of an alpha-L-glutamate or alpha-L-glutamic homopolymer.

13. Formulation according to claim 6, **characterized in that** the PO consists of an alpha-L-aspartate or alpha-L-aspartic homopolymer.

14. Formulation according to claim 6, **characterized in that** the PO consists of an alpha-L-aspartate/alpha-L-glutamate or alpha-L-aspartic/alpha-L-glutamic copolymer.

15. Formulation according to claim 14, **characterized in that**, in the PO, the distribution of the aspartic and/or glutamic units carrying grafts containing at least one HG unit is such that the resulting polymer is either random or of the block type or of the multiblock type.

16. Formulation according to claim 1, **characterized in that** the molecular weight of the PO is between 2000 and 100,000 g/mol and preferably between 5000 and 40,000 g/mol.

17. Formulation according to claims 7 and 9, **characterized in that** the hydrophobic radical R⁶ of the graft of the PO is derived from an alcohol precursor formed of tocopherol, and **in that**:
◆ 1% ≤ [n/(n + m)] x 100 ≤ 10%,
◆ preferably 3.5% ≤ [n/(n + m)] x 100 ≤ 7.5%,
◆ n + m varies from 100 to 400 and preferably between 120 and 300.

18. Formulation according to claims 7 and 9, **characterized in that** the hydrophobic radical R⁶ of the graft of the PO is derived from an alcohol precursor formed of cholesterol:
◆ 1% ≤ [n/(n + m)] x 100 ≤ 10%,
◆ preferably 3.5% ≤ [n/(n + m)] x 100 ≤ 6.5%,
◆ n + m varies from 100 to 400 and preferably between 120 and 300.

19. Formulation according to claim 17 or 18, **characterized in that** the concentration of polymer [PO] is between 15 and 50 mg/ml.

20. Formulation according to any one of claims 1 to 19, **characterized in that** its viscosity is less than or equal to 5 Pa.s at 20°C.

21. Formulation according to any one of claims 1 to 20, **characterized in that** its weight fraction of interleukin(s) not associated with the submicronic particles [non-associated interleukin(s)], in %, is such that:
○ [non-associated interleukin(s)] ≤ 1,
○ preferably [non-associated interleukin(s)] ≤ 0.5,
○ most preferably [non-associated interleukin(s)] ≤ 0.1.

22. Formulation according to any one of claims 1 to 21, **characterized in that** the interleukin is interleukin 2.

23. Formulation according to any one of claims 1 to 22, **characterized in that** the additional active principle(s) other than the interleukin(s) is (are) a protein, a glycoprotein, a protein bonded to one or more polyalkylene glycol chains [preferably polyethylene glycol (PEG) chains: "PEGylated protein"], a polysaccharide, a liposaccharide, an oligonucleotide, a polynucleotide or a peptide, this (these) additional active principle(s) preferably being selected from haemoglobins, cytochromes, albumins, interferons, cytokines, antigens, antibodies, erythropoietin, insulin, growth hormones, factors VIII and IX, haemopoiesis stimulating factors, and mixtures thereof.

24. Formulation according to any one of claims 1 to 23, **characterized in that** it is injectable by the parenteral, subcutaneous, intramuscular, intradermal, intraperitoneal or intracerebral route or into a tumour.

25. Formulation according to any one of claims 1 to 24, **characterized in that** it is intended for the preparation of drugs, particularly for administration by the parenteral, subcutaneous, intramuscular, intradermal, intraperitoneal or intracerebral route or into a tumour, or by the oral, nasal, vaginal or ocular route.

26. Process for the preparation of drugs, particularly for administration by the parenteral, subcutaneous, intramuscular, intradermal, intraperitoneal or intracerebral route or into a tumour, or by the oral, nasal, vaginal or ocular route, **characterized in that** it consists essentially in using at least one formulation according to any one of claims 1 to 25.

27. Derived product, **characterized in that** it comprises submicronic particles formed of non-covalent PO/AP associations as defined in claim 1, and **in that** it is obtained from the formulation according to any one of claims 1 to 25.

28. Derived product according to claim 27, **characterized in that** it consists of a powder or a gel.

29. Process for the preparation of the formulation according to any one of claims 1 to 26, **characterized in that** it consists essentially in:
◆ taking a colloidal suspension of nanoparticles of at least one PO,
◆ mixing this colloidal suspension of nanoparticles of PO with at least one interleukin (and one or more other possible active principles), preferably in aqueous solution,
◆ optionally adding at least one excipient,
◆ adjusting the pH and/or the osmolarity if necessary, and
◆ optionally filtering the resulting suspension.

30. Process according to claim 29, **characterized in that** the AP is (are) in the form of an aqueous suspension or solution for mixing with the colloidal suspension of nanoparticles of PO.

31. Process for the preparation of the formulation according to any one of claims 1 to 25, **characterized in that** it consists essentially in:
◆ taking a powder of at least one polymer PO,
◆ mixing this powder with an aqueous suspension or solution of at least one interleukin (and one or more other possible active principles), preferably in aqueous solution,
◆ optionally adding at least one excipient,
◆ adjusting the pH and/or the osmolarity if necessary, and
◆ optionally filtering the resulting suspension.

32. Process for the preparation of the formulation according to any one of claims 1 to 25, **characterized in that** it consists essentially in:
◆ taking a powder produced by drying the liquid formulation according to any one of claims 1 to 25,
◆ mixing this powder with an aqueous liquid medium, preferably with stirring,
◆ optionally adding at least one excipient,
◆ adjusting the pH and/or the osmolarity if necessary, and
◆ optionally filtering the resulting suspension.

33. Process for the preparation of a powder derived from the formulation according to any one of claims 1 to 25, **characterized in that** said powder is obtained by drying the formulation according to any one of claims 1 to 25.

## Patentansprüche

1. Flüssige pharmazeutische Formulierung zur verzögerten Freisetzung von Interleukin(en), wobei die Formulierung Folgendes umfasst: mindestens ein Interleukin, gegebenenfalls mindestens einen anderen Wirkstoff (WS), vorzugsweise in wässriger Lösung, und eine wässrige kolloidale Suspension mit niedriger Viskosität auf der Basis von Submikron-Partikeln aus wasserlöslichem, biologisch abbaubarem Polymer (PO), das aus der Gruppe ausgewählt ist, bestehend aus Polyaminosäuren, Polysacchariden, Gelatinen oder ihren Gemischen, und das hydrophobe Gruppen (HG) trägt, wobei die Partikel mit dem Interleukin und gegebenenfalls dem anderen Wirkstoff (WS) nicht-kovalent assoziiert sind, **dadurch gekennzeichnet, dass**
◆ das Dispergiermedium der Suspension im Wesentlichen aus Wasser besteht,
◆ ihre Konzentration an [PO] auf einen Wert [PO] ≥ 0,9.C1 derart festgelegt wird, dass die pharmazeutische Formulierung auf parenteralem Weg injiziert werden und anschließend in vivo ein geliertes Depot bilden kann, wobei C1 für die Konzentration der "induzierten Gelierung" der PO-Partikel steht, wie sie in einem GI-Test, wie in der Beschreibung beschrieben, gemessen wird, wobei die Bildung eines gelierten Depots:
○ einerseits zumindest teilweise durch mindestens ein in vivo vorhandenes physiologisches Protein ausgelöst wird
○ und es andererseits gestattet, die Dauer der Freisetzung des Interleukins und gegebenenfalls des WS in vivo auf jenseits von 24 Std. nach der Verabreichung zu verlängern und zu steuern,
◆ sie unter den Injektionsbedingungen flüssig ist
◆ und ebenfalls flüssig ist bei der physiologischen Temperatur und/oder dem physiologischen pH und/oder in Anwesenheit von:
* einem physiologischen Elektrolyten in physiologischer Konzentration
* und/oder mindestens einem oberflächenaktiven Mittel.

2. Flüssige pharmazeutische Formulierung zur verzögerten Freisetzung von Interleukin(en) und gegebenenfalls (einem) anderen Wirkstoff(en) -WS-, wobei die Formulierung:
○ unter Umgebungsatmosphäre flüssig ist,
○ ebenfalls flüssig ist bei der physiologischen Temperatur und/oder dem physiologischen pH und/oder in Anwesenheit von:
* einem physiologischen Elektrolyten in physiologischer Konzentration
* und/oder mindestens einem oberflächenaktiven Mittel,
○ und Folgendes umfasst: mindestens ein Interleukin, gegebenenfalls mindestens einen anderen Wirkstoff (WS), vorzugsweise in wässriger Lösung, und eine wässrige kolloidale Suspension mit niedriger Viskosität auf der Basis von Submikron-Partikeln aus wasserlöslichem, biologisch abbaubarem Polymer (PO), das aus der Gruppe ausgewählt ist, bestehend aus Polyaminosäuren, Polysacchariden, Gelatinen oder ihren Gemischen, und das hydrophobe Gruppen (HG) trägt, wobei die Partikel mit dem Interleukin und gegebenenfalls dem anderen Wirkstoff (WS) nicht-kovalent assoziiert sind und das Dispergiermedium der Suspension im Wesentlichen aus Wasser besteht,
**dadurch gekennzeichnet, dass** ihre Konzentration an [PO] auf einen Wert festgelegt wird, der genügend erhöht ist, dass die Bildung eines gelierten Depots in vitro in Anwesenheit mindestens eines Proteins ermöglicht wird, wobei die Konzentration an [PO] derart ist, dass [PO] ≥ 0,9.C1, wobei C1 für die Konzentration der "induzierten Gelierung" der PO-Partikel steht, wie sie in einem GI-Test, wie in der Beschreibung beschrieben, gemessen wird.

3. Formulierung nach einem der vorhergehenden Ansprüche, **dadurch gekennzeichnet, dass** ihre Konzentration an [PO]:
■ 20.C1 ≥ [PO] ≥ C1
■ und noch besser 10.C1 ≥ [PO] ≥ C1
beträgt.

4. Formulierung nach einem der vorhergehenden Ansprüche, **dadurch gekennzeichnet, dass** die hydrophoben modifizierten Polymere PO aus der Gruppe ausgewählt werden, aus den Polysacchariden aus der Gruppe der Pullulane, der Chitosane und der Mucopolysaccharide besteht.

5. Formulierung nach einem der vorhergehenden Ansprüche, **dadurch gekennzeichnet, dass** die hydrophoben Gruppen (HG) sich seitlich an der Kette befinden.

6. Formulierung nach einem der vorhergehenden Ansprüche, **dadurch gekennzeichnet, dass** das Polymer PO eine Polyaminosäure ist, die aus Asparaginsäure-Einheiten und/oder Glutaminsäure-Einheiten gebildet ist, wobei zumindest ein Teil dieser Einheiten Pfropfungen trägt, die mindestens eine hydrophobe Gruppe (HG) tragen.

7. Formulierung nach Anspruch 6, **dadurch gekennzeichnet, dass** das (oder die) PO durch die folgende allgemeine Formel (I) definiert ist (oder sind): 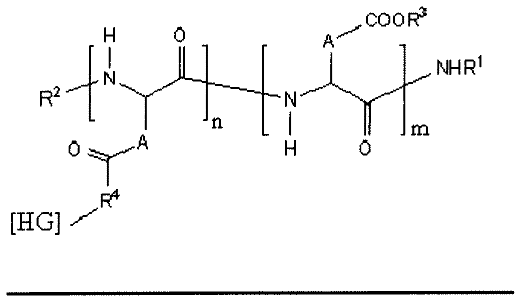 worin:
■ R¹ für H, ein gerades C2- bis C10- oder verzweigtes C3- bis C10-Alkyl, Benzyl, eine endständige Aminosäure-Einheit oder -R⁴-[HG] steht,
■ R² für H, eine gerade C2- bis C10- oder verzweigte C3- bis C10-Acylgruppe, ein Pyroglutamat oder -R⁴-[HG] steht,
■ R³ für H oder eine kationische Einheit steht, die vorzugsweise aus der Gruppe ausgewählt ist, umfassend:
- Metallkationen, die vorteilhafterweise aus der Untergruppe ausgewählt sind, umfassend: Natrium, Kalium, CalC1um, Magnesium,
- organische Kationen, die vorteilhafterweise aus der Untergruppe ausgewählt sind, umfassend:
• Kationen auf Basis eines Amins,
• Kationen auf Basis eines Oligoamins,
• Kationen auf Basis eines Polyamins (wobei Polyethylenimin besonders bevorzugt ist),
• Kationen auf Basis einer Aminosäure (von Aminosäuren), die vorteilhafterweise aus der Klasse ausgewählt sind, die Kationen auf der Basis von Lysin oder Arginin umfasst,
- oder kationische Polyaminosäuren, die vorteilhafterweise aus der Untergruppe ausgewählt sind, die Polylysin oder Oligolysin umfasst,
■ R⁴ für eine direkte Bindung oder einen "Spacer" auf Basis von 1 bis 4 Aminosäure-Einheiten steht,
■ A unabhängig für einen Rest -CH₂- (Asparaginsäure-Einheit) oder -CH₂-CH₂- (Glutaminsäure-Einheit) steht,
■ n/(n+m) als das molare Pfropfungsverhältnis definiert ist und von 0,5 bis 100 Mol-% variiert,
■ n/(n+m) als das molare Pfropfungsverhältnis definiert ist, dessen Wert ausreichend niedrig ist, dass PO, gelöst in Wasser bei pH 7 und 25°C, eine kolloidale Suspension von Submikron-Partikeln von PO bildet, vorzugsweise liegt n/(n+m) zwischen 1 bis 25 Mol-% und noch stärker bevorzugt zwischen 1 bis 15 Mol-%,
■ n + m von 10 bis 1000, vorzugsweise zwischen 50 und 300, variiert,
■ HG für eine hydrophobe Gruppe steht.

8. Formulierung nach Anspruch 6, **dadurch gekennzeichnet, dass** das (oder die) PO einer der folgenden allgemeinen Formeln (II), (III) und (IV) entspricht/entsprechen: 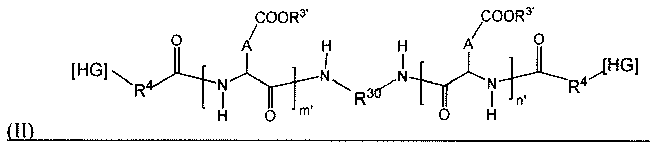 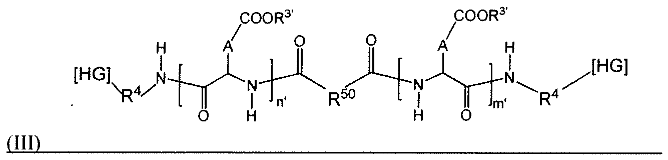 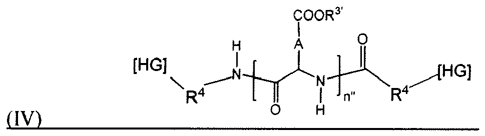 in denen:
■ HG für eine hydrophobe Gruppe steht,
■ R³⁰ eine gerade C2- bis C6-Alkylgruppe ist,
■ R^{3'} für H oder eine kationische Einheit steht, die vorzugsweise aus der Gruppe ausgewählt ist, umfassend:
- Metallkationen, die vorteilhafterweise aus der Untergruppe ausgewählt sind, umfassend: Natrium, Kalium, CalC1um, Magnesium,
- organische Kationen, die vorteilhafterweise aus der Untergruppe ausgewählt sind, umfassend:
• Kationen auf Basis eines Amins,
• Kationen auf Basis eines Oligoamins,
• Kationen auf Basis eines Polyamins (wobei Polyethylenimin besonders bevorzugt ist),
• Kationen auf Basis einer Aminosäure (von Aminosäuren), die vorteilhafterweise aus der Klasse ausgewählt sind, die Kationen auf der Basis von Lysin oder Arginin umfasst,
- oder kationische Polyaminosäuren, die vorteilhafterweise aus der Untergruppe ausgewählt sind, die Polylysin oder Oligolysin umfasst,
■ R⁵⁰ für eine C2- bis C6-Alkyl-, -Dialkoxy- oder Diamingruppe steht,
■ R⁴ für eine direkte Bindung oder einen "Spacer" auf Basis von 1 bis 4 Aminosäure-Einheiten steht,
■ A unabhängig für einen Rest -CH₂- (Asparaginsäure-Einheit) oder -CH₂-CH₂- (Glutaminsäure-Einheit) steht,
■ n' + m' oder n " als Polymerisierungsgrad definiert ist und von 10 bis 1000, vorzugsweise zwischen 50 und 300, variiert.

9. Formulierung nach Anspruch 7 oder 8, **dadurch gekennzeichnet, dass** die n Gruppen HG des PO jeweils unabhängig voneinander für einen einwertigen Rest der folgenden Formel stehen: 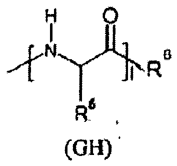 worin:
- R⁵ für Methyl (Alanin), Isopropyl (Valin), Isobutyl (LeuC1n), sek.-Butyl (IsoleuC1n), Benzyl (Phenylalanin) steht,
- R⁶ für einen hydrophoben Rest steht, der 6 bis 30 Kohlenstoffatome trägt,
- 1 von 0 bis 6 variiert.

10. Formulierung nach Anspruch 9, **dadurch gekennzeichnet, dass** sämtliche oder ein Teil der hydrophoben Reste R⁶ der PO unabhängig aus der Gruppe der folgenden Reste ausgewählt ist/sind:
■ einem geraden oder verzweigten Alkoxy mit 6 bis 30 Kohlenstoffatomen, das mindestens ein Heteroatom (vorzugsweise O und/oder N und/oder S) und/oder mindestens eine Ungesättigtheit tragen kann,
■ einem Alkoxy mit 6 bis 30 Kohlenstoffatomen, das einen oder mehrere kondensierte Carbozyklen besitzt und gegebenenfalls mindestens eine Ungesättigtheit und/oder mindestens ein Heteroatom (vorzugsweise O und/oder N und/oder S) enthält,
■ einem Alkoxyaryl oder einem Aryloxyalkyl mit 7 bis 30 Kohlenstoffatomen, das mindestens eine Ungesättigtheit und/oder mindestens ein Heteroatom (vorzugsweise O und/oder N und/oder S) tragen kann.

11. Formulierung nach Anspruch 9 oder 10, **dadurch gekennzeichnet, dass** der hydrophobe Rest R⁶ der Pfropfung des PO von einer Alkohol-Vorstufe stammt, die aus der Gruppe ausgewählt ist, umfassend: Octanol, Dodecanol, Tetradecanol, Hexadecanol, Octadecanol, Oleylalkohol, Tocopherol oder Cholesterin.

12. Formulierung nach Anspruch 6, **dadurch gekennzeichnet, dass** das PO aus einem Homopolymer von alpha-L-Glutamat oder alpha-L-Glutaminsäure besteht.

13. Formulierung nach Anspruch 6, **dadurch gekennzeichnet, dass** das PO aus einem Homopolymer von alpha-L-Aspartat oder alpha-L-Asparaginsäure besteht.

14. Formulierung nach Anspruch 6, **dadurch gekennzeichnet, dass** das PO aus einem Copolymer von alpha-L-Aspartat/alpha-L-Glutamat oder alpha-L-Asparaginsäure/- alpha-L-Glutaminsäure besteht.

15. Formulierung nach Anspruch 14, **dadurch gekennzeichnet, dass** in dem PO die Verteilung der Asparaginsäure- und/oder Glutaminsäure-Einheiten, die Pfropfungen mit mindestens einem HG-Motiv tragen, derart ist, dass das so hergestellte Polymer entweder zufallsgemäß oder vom Block- oder Multiblock-Typ ist.

16. Formulierung nach Anspruch 1, **dadurch gekennzeichnet, dass** die Molmasse des PO zwischen 2000 und 100000 g/mol und vorzugsweise zwischen 5000 und 40000 g/mol liegt.

17. Formulierung nach den Ansprüchen 7 und 9, **dadurch gekennzeichnet, dass** der hydrophobe Rest R⁶ der Pfropfung des PO von einer Alkohol-Vorstufe stammt, die von Tocopherol gebildet wird, und dadurch, dass:
◆ 1% ≤ [n/(n + m)] x 100 ≤ 10%
◆ vorzugsweise 3,5% ≤ [n/(n + m)] x 100 ≤ 7,5%
◆ n + m von 100 bis 400, vorzugsweise zwischen 120 und 300, variiert.

18. Formulierung nach den Ansprüchen 7 und 9, **dadurch gekennzeichnet, dass** der hydrophobe Rest R⁶ der Pfropfung des PO von einer Alkohol-Vorstufe stammt, die von Cholesterin gebildet wird:
◆ 1% ≤ [n/(n + m)] x 100 ≤ 10%
◆ vorzugsweise 3,5% ≤ [n/(n + m)] x 100 ≤ 6,5%
◆ n + m von 100 bis 400, vorzugsweise zwischen 120 und 300, variiert.

19. Formulierung nach Anspruch 17 oder 18, **dadurch gekennzeichnet, dass** die Konzentration an Polymer [PO] zwischen 15 und 50 mg/ml beträgt.

20. Formulierung nach einem der Ansprüche 1 bis 19, **dadurch gekennzeichnet, dass** ihre Viskosität bei 20°C kleiner oder gleich 5 Pa.s ist.

21. Formulierung nach einem der Ansprüche 1 bis 20, **dadurch gekennzeichnet, dass** ihr Massenanteil an Interleukin(en), das (die) nicht mit den Submikron-Partikeln assoziiert ist (sind), [nicht-assoziierte(s) Interleukin(e)] in Gew.-% derart ist, dass:
○ [nicht-assoziierte(s) Interleukin(e)] ≤ 1
○ vorzugsweise [nicht-assoziierte(s) Interleukin(e)] ≤ 0,5
○ und noch stärker bevorzugt [nicht-assoziierte(s) Interleukin(e)] ≤ 0,1.

22. Formulierung nach einem der Ansprüche 1 bis 21, **dadurch gekennzeichnet, dass** es sich bei dem Interleukin um Interleukin 2 handelt.

23. Formulierung nach einem der Ansprüche 1 bis 22, **dadurch gekennzeichnet, dass** der (oder die) zusätzliche(n) andere(n) Wirkstoff(e) als das (oder die) Interleukin(e) ein Protein, ein Glykoprotein, ein Protein, das an eine oder mehrere Polyalkylenglykol-Ketten [vorzugsweise Polyethylenglykol (PEG): "PEGlyiertes Protein"] gebunden ist, ein Polysaccharid, ein Liposaccharid, ein Oligonukleotid, ein Polynukleotid oder ein Peptid ist, wobei dieser (oder diese) zusätzliche(n) Wirkstoff(e) vorzugsweise aus Hämoglobinen, Cytochromen, Albuminen, Interferonen, Cytokinen, Antigenen, Antikörpern, Erythropoetin, Insulin, Wachstumshormonen, den Faktoren VIII und IX, die Hämatopoese stimulierenden Faktoren oder deren Gemischen ausgewählt ist (sind).

24. Formulierung nach einem der Ansprüche 1 bis 23, **dadurch gekennzeichnet, dass** sie auf parenteralem, subkutanem, intramuskulärem, intradermalem, intraperitonealem, intrazerebralem Weg oder in einen Tumor injizierbar ist.

25. Formulierung nach einem der Ansprüche 1 bis 24, **dadurch gekennzeichnet, dass** sie für die Herstellung von Arzneimitteln, insbesondere zur parenteralen, subkutanen, intramuskulären, intradermalen, intraperitonealen, intrazerebralen Verabreichung oder zur Verabreichung in einen Tumor, insbesondere auf oralem, nasalem, vaginalem oder okularem Weg, bestimmt ist.

26. Verfahren zur Herstellung von Arzneimitteln, insbesondere zur parenteralen, subkutanen, intramuskulären, intradermalen, intraperitonealen, intrazerebralen Verabreichung oder zur Verabreichung in einen Tumor, insbesondere auf oralem, nasalem, vaginalem oder okularem Weg, **dadurch gekennzeichnet, dass** es im Wesentlichen aus der Verwendung mindestens einer Formulierung nach einem der Ansprüche 1 bis 25 besteht.

27. Abgeleitetes Produkt, **dadurch gekennzeichnet, dass** es Submikron-Partikel umfasst, die von nicht-kovalenten PO/WS-Assoziationen nach Anspruch 1 gebildet werden, und dadurch, dass es ausgehend von der Formulierung nach einem der Ansprüche 1 bis 25 erhalten wird.

28. Abgeleitetes Produkt nach Anspruch 27, **dadurch gekennzeichnet, dass** es aus einem Pulver oder einem Gel besteht.

29. Verfahren zur Herstellung der Formulierung nach einem der Ansprüche 1 bis 25, **dadurch gekennzeichnet, dass** man im Wesentlichen:
◆ eine kolloidale Suspension von Nanopartikeln mindestens eines PO verwendet,
◆ diese kolloidale Suspension von PO-Nanopartikeln mit mindestens einem Interleukin (und einem oder mehreren anderen optionalen Wirkstoff(en)), vorzugsweise in wässriger Lösung, mischt,
◆ gegebenenfalls mindestens einen ExClpienten hinzufügt,
◆ bei Bedarf den pH und/oder die Osmolarität einstellt und
◆ gegebenenfalls die so erhaltene Suspension filtriert.

30. Verfahren nach Anspruch 29, **dadurch gekennzeichnet, dass** der (oder die) WS die Form einer Suspension oder einer wässrigen Lösung für das Mischen mit der kolloidalen Suspension von PO-Nanopartikeln hat (haben).

31. Verfahren zur Herstellung der Formulierung nach einem der Ansprüche 1 bis 25, **dadurch gekennzeichnet, dass** man im Wesentlichen:
◆ ein Pulver von mindestens einem Polymer PO verwendet,
◆ dieses Pulver mit einer Suspension oder wässrigen Lösung von mindestens einem Interleukin (und einem oder mehreren anderen optionalen Wirkstoff(en)), vorzugsweise in wässriger Lösung, mischt,
◆ gegebenenfalls mindestens einen ExClpienten hinzufügt,
◆ bei Bedarf den pH und/oder die Osmolarität einstellt und
◆ gegebenenfalls die so erhaltene Suspension filtriert.

32. Verfahren zur Herstellung der Formulierung nach einem der Ansprüche 1 bis 25, **dadurch gekennzeichnet, dass** man im Wesentlichen:
◆ ein Pulver verwendet, das aus der Trocknung der flüssigen Formulierung nach einem der Ansprüche 1 bis 25 hervorgegangen ist,
◆ dieses Pulver mit einem wässrigen flüssigen Medium, vorzugsweise unter Rühren, mischt,
◆ gegebenenfalls mindestens einen ExClpienten hinzufügt,
◆ bei Bedarf den pH und/oder die Osmolarität einstellt und
◆ gegebenenfalls die so erhaltene Suspension filtriert.

33. Verfahren zur Herstellung eines Pulvers, das von der Formulierung nach einem der Ansprüche 1 bis 25 stammt, **dadurch gekennzeichnet, dass** das Pulver durch Trocknung der Formulierung nach einem der Ansprüche 1 bis 25 erhalten wird.
